# EUROPÄISCHE PATENTSCHRIFT

(11) **EP 1 523 365 B1**
(45) Veröffentlichungstag und Bekanntmachung des Hinweises auf die Patenterteilung: **11.10.2017**
(21) Anmeldenummer: 03764928.2
(22) Anmeldetag: 30.06.2003
(51) Int. Cl.: A61M 15/00

(54) **HANDBETÄTIGBARER INHALATOR FÜR PULVERFÖRMIGE SUBSTANZEN**
MANUAL INHALATOR FOR POWDERED SUBSTANCES
INHALATEUR À ACTIONNEMENT MANUEL POUR SUBSTANCES PULVÉRULENTES

(30) Priorität: 22.07.2002 DE 10233150
(43) Veröffentlichungstag der Anmeldung: 20.04.2005
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim
(86) Internationale Anmeldenummer: PCT/EP2003/006942
(87) Internationale Veröffentlichungsnummer: WO 2004/009168

(56) Entgegenhaltungen:
- EP-A- 0 652 022
- WO-A-01/15760

## Beschreibung

Die Erfindung bezieht sich auf einen handbetätigbaren Inhalator für pulverförmige Substanzen, insbesondere medikamentöse Substanzen, bei welchem sich bei der Handbetätigung eine bestimmte Ausgabemenge aus einer Substanzen-Vorratsmenge in einer Dosierkammer vor einem Austragskanal abteilt, zwecks luftgetragener Ausgabe aus einer Mundstücköffnung am Ende des Austragskanals, wobei ein den Ausgabe-Luftstrom erzeugender Kolben mit einem Hohlraum seines Schaftes eine Substanzen-Vorratskammer und die Dosierkammer bildet, wobei ein beim Rückhub des Kolbens erzeugter Unterdruck zur Substanzen-Vorratsmenge hin öffnet, und wobei weiter der Boden der Dosierkammer von einer luftdurchlässigen Membran gebildet ist.

Aus der DE-PS 44 15 462 und der EP 0 561 838 sind Applikationshilfen für pulverförmige Substanzen in Form eines auf eine Mund-Saug-Entleerung hin konzipierten Inhalators bekannt. Die Saugkraft fällt, je nach Konstitution des Benutzers, individuell meist hochgradig unterschiedlich aus.

Aus der WO 90/07351 geht ein Inhalator hervor, bei dem die Entleerung der Dosierkammer durch eine Pumpen/Zylinder-Einheit bewirkt wird. Weiter ist auch auf die EP 0 652 022 A1 zu verweisen.

Das Verabreichen pulverförmiger Substanzen, insbesondere von Medikamenten, erfordert neben einer feinen Verteilung im transportierenden Luftstrom auch, dass dieser immer gleich kraftvoll austritt und möglichst stärker ist als ein Patient üblicherweise saugt bzw. saugen kann. Nur so kann das pulverförmige Gut auch sicher an den Zielort gelangen. Dabei ist es des Weiteren erforderlich, dass die Mengen exakt reproduzierbar sind. Das setzt unter anderem auch voraus, dass die pulverförmige Substanz, d.h. die Substanzen-Vorratsmenge, nicht blockt. Im Hinblick auf Pumpenkolben-Lösungen gilt es ferner, die unangenehme Schlagwirkung zu beherrschen. Es soll zwar ein kraftvoller transportierender Luftstrom aufgebracht werden können, der darf aber nicht übermäßig sein.

Die vorliegende Erfindung geht vom Gegenstand der WO 01/15760 A1 (DE 199 63 946 C2) aus.

Aufgabe der Erfindung ist es, einen gattungsgemäßen Inhalator im Sinne der geschilderten Ziele bzw. Vorgaben funktionssicher und gebrauchsvorteilhaft auszubilden.

Diese Aufgabe ist bei einem handbetätigten Inhalator mit den Merkmalen des Anspruches 1 gelöst, wobei in Ergänzung der Merkmalskombination darauf abgestellt ist, dass das aus der Kolbenbewegung resultierende Luftstrom-Volumen mehr als das Hundertfache des Volumens der Dosierkammer beträgt, aber weniger als das Sechshundertfache. Das führt zu einem kraftvollen, nicht schlagenden, die pulverförmige Substanz fein verteilt transportierenden Luftstrom. Die Pulverpartikel-Dichte ist hervorragend auf das Luftstrom-Volumen abgestimmt. Zugleich sind stärkere mechanische Belastungen der luftdurchlässigen Membran vermieden. Die Lebensdauer eines solchen Inhalators ist erhöht. Bevorzugt beträgt das Luftstrom-Volumen das Vierhundertfache des Volumens der Dosierkammer. 10 mg an pulverförmiger Substanz werden in einen ca. 4 ml Luftstrom aufgenommen. Das Volumen der Dosierkammer beträgt dabei 10 mm³.

Hinzu kommen noch beibehaltene Merkmale und Eigenschaften der gattungsgemäßen Grundversion. So ist die pulverförmige Substanz in einem sich bewegenden Bauteil des Inhalators untergebracht, nämlich dem Schaft des Kolbens.

Die Substanz bewegt sich also stets mit. Neben dieser gleichsam mechanischen Rütteleinrichtung (sowohl die Hin- als auch die Rüttelbewegung ist genutzt) wird eine Rutschbewegung der pulverförmigen Substanz durch das jeweilige Nachfüllen der Dosierkammer erzeugt.

Ein Ausgabe-Druckluftstrom geht über den pulvergefüllten Hohlraum der Dosierkammer und ein Luftunterdruck öffnet die Kammer. Verblockung der pulverförmigen Substanz ist so praktisch ausgeschlossen. Der Luftstrom wird stets gleich stark aufgebracht. Nutzen lässt sich dabei der relativ große Kolbenquerschnitt in Bezug auf den relativ querschnittskleineren Austragskanal, dem die strömungsdämmende Barriere der luftdurchlässigen Membran vorgeschaltet ist.

Weiter wird vorgeschlagen, dass der Schaft an einem dem Kolben gegenüberliegenden Ende die Mundstücköffnung bildet. Letztere lässt sich in Bezug auf den Luftstrom optimal formen.

Weiter erweist es sich als günstig, dass der Austragskanal als sich im Zentrum des Schaftes des unter Federspannung stehendem Kolbens erstreckendes Schaft-Innenrohr gestaltet ist, unter dessen kolbenseitigem Ende sich die Ausgabemenge sammelt. Ein solches Zentralsystem schafft einen Zugangsringraum für die pulverförmige Substanz, was auch wieder förderlich für die erstrebte Präzision in der Dosierung ist.

Weiter erweist es sich als günstig, dass der handbetätigte Kolben-Federspannhub der Ausgabehub ist und sich die Ausgabemenge beim federveranlassten Rückhub des Kolbens sammelt. Das Bilden der Ausgabemenge geschieht so praktisch automatisch. Es steht bis zum Ende immer eine ausgabebereite Menge zur Verfügung.

Eine vorteilhafte Dosierkammer wird erreicht, wenn sich die Ausgabemenge in einer Vertiefung im Boden der Substanzen-Vorratskammer sammelt und der obere Rand der Vertiefung zwischen einer Dicht- und Öffnungsstellung des Schaft-Innenrohres wechselt. Der Rand der Vertiefung der Dosierkammer kann zugangssperrend unmittelbar gegen das Schaft-Innenrohr treten oder mittelbar. Er stellt sich vorteilhafterweise aufgrund der inneren Elastizität zurück in die Dichtstellung.

Der Übertritt in die Öffnungsstellung resultiert aus einer elastischen Verlagerung dieses Bodens der Substanzen-Vorratskammer aufgrund des beim Rückhub des Kolbens hinter bzw. unter diesem auftretenden Unterdrucks. Es fällt dabei pulverförmige Substanz in die Dosierkammer. Ein Saugstrom füllt die Substanzen-Vorratskammer oberhalb des Füllspiegels um das entsprechende Volumen wieder auf, welches in die Dosierkammer abgewandert ist.

Eine luftdurchlässige Abdeckung eines Loches im Boden der Substanzen-Vorratskammer soll einerseits möglichst leicht Luft durchlassen zum Ausstoßen des Pulvers, andererseits aber in umgekehrter Richtung so sein, dass der Unterdruck den Rand der Dosierkammer von seinem Dichtsitz genügend abhebt. Hier kann ein Filterblättchen eingesetzt werden, bestehend aus Vlies oder auch gewebtem Material. Die Porenweite liegt bei einem µ. Das Pulver kann so selbst nicht durchtreten.

Eine erste dünne Schicht des eingefallenen Pulvers schließt die Poren eines solchen Materials. In baulich vorteilhafter Weise sind der Boden und die Vertiefung der Substanzen-Vorratskammer von einer elastischen topfförmigen Membran gebildet, deren Topf-Innenwand ein Einsatzteil trägt, auf dessen oberem Rand das Schaft-Innenrohr mit einer Gegenschließfläche dichtend aufsetzt. Dosiermenge und Substanzen-Vorratsmenge werden durch eine solche topfförmige Membran separiert, wenn die Membran geschlossen ist.

Weiter wird vorgeschlagen, dass der Kolben eine entgegen der Richtung des Rückhubes weisende Kolbenlippe besitzt, die gleitend an der Innenwand des Zylinders anliegt. Ein so ermöglichtes Hinterströmen der Kolbenlippe sichert gleichmäßigen Unterdruck beim Rückhub des Kolbens. Das schützt die Abdeckung bzw. Membran vor einem Einreißen oder dergleichen.

Ein vorteilhaftes Merkmal besteht überdies darin, dass die Durchlässigkeit der Abdeckung in Relation zur Feinkörnigkeit des Pulvers so ist, dass die nach erster Öffnungsbewegung des Bodens auf diesen fallende dünnschichtige Pulvermenge die Luftdurchlässigkeit in Öffnungsrichtung beseitigt.

Sodann besteht ein vorteilhaftes Merkmal darin, dass das Schaft-Innenrohr sich bis kurz vor die Mündungsöffnung erstreckt und zur Wand des umgebenden Schaftmaterials einen Lufteinströmkanal freilässt, der bis in die Substanzen-Vorratskammer reicht. Hierüber entsteht eine die pulverförmige Substanz auch aufmischende, ziehende Luftströmung der Unterdruckquelle. Dabei ist vorgesehen, dass im oberen Bereich der Substanzen-Vorratskammer eine für die Einströmluft durchlässige Decke ausgebildet ist, die das Schaft-Innenrohr beiderseits abstützend kreuzt und ein zentrales Loch fluchtend zum Austragskanal besitzt. Der Austragskanal ist so durchgehend offen; das Umfeld des Schaft-Innenrohres lässt Luft passieren, hält aber die pulverförmige Substanz zurück.

Weiter wird vorgeschlagen, dass der Austragskanal sich in Strömungsrichtung an der Ausgabenmengen-Sammelstelle trichterförmig verjüngt. Das vergrößert das Einzugsgebiet für die Substanz mit zentrierender wie beschleunigender Wirkung auf den Tragstrom.

Um zu verhindern, dass bei dem Sammeln der pulverförmigen Substanz zu einer dosierten Ausgabemenge Falschluft als Behinderung auftritt, ist eine Sperre vorgesehen. Die besteht darin, dass vor dem zentralen Loch ein Ventilkörper angeordnet ist, der in Austragsrichtung öffnet. Das Verformen des die Dosierkammer bildenden Hohlraumes durch den Unterdruck beim Übertritt in die Öffnungsstellung der Dosierkammer schafft einen Freiraum unterhalb des Schaft-Innenrohres, der dann nur mit einem eng begrenzten Luftvolumen in Verbindung tritt, welches Luftvolumen vom Dichtsitzrand bis zur Ventilsperre reicht.

Überdies ergibt sich eine vorteilhafte Betätigungsweise durch eine Ansprechschwelle für die handbetätigte Kolbenverlagerung. Die Ansprechschwelle lässt einen bestimmten Betätigungsdruck entstehen. Der ist gut beherrschbar gewählt. Bei Zusammenbrechen des überwindbaren Widerstandes der Ansprechschwelle wird so eine beschleunigte Verlagerung des Kolbens relativ zum Gehäuse des handbetätigbaren Inhalators erreicht. Das verkörpert sich baulich im Einzelnen dadurch, dass die Ansprechschwelle von einem Ringkörper des Kolbenschaftes rückseitig der Kolbenmanschette gebildet ist, welcher Ringkörper in eine Rastnut der dem Kolben zugehörigen Zylinderwand einrastet. Einschließlich des widerstands-mitbildenden Ringkörpers beträgt der Öffnungsdruck ca. 2,5 kg. Bezüglich des Ringkörpers handelt es sich zweckmäßig um einen ovalen Federring, der querschnittsmäßig im lichten Durchmesser des Zylinders im nicht rastgenuteten Bereich unterkommt.

Eine vorteilhafte Weiterbildung des Inhalators besteht darin, dass die Dosierkammer von einem endseitigen Erbreiterungsbereich des auf die luftdurchlässige Membran bzw. Abdeckung des Bodens aufsetzenden und beim Federrückhub abhebenden Austragskanals gebildet ist. Realiter wird die Öffnungsbewegung natürlich vom abhebenden Boden ausgeführt. Das gleichflächige Aufsetzen auf die membranartig gespannte Abdeckung lässt eine Dosierkammer erreichen, die nicht mehr, wenn auch nur partiell, durch den Endabschnitt des den Austragskanal stellenden Schaft-Innenrohr besetzt ist, sondern bloß hütchenartig begrenzt wird. Das vereinfacht den Aufbau. Auch hier sind die Verhältnisse gemäß Kennzeichen des Anspruches 1 angewandt.

Weiter wird vorgeschlagen, dass die Umfassungswand der Dosierkammer sich in Austragsrichtung kegelförmig erweitert. Das schafft ein sammelfreudiges Umfeld für die Dosierkammer in Richtung der Dosierkammer-Vertiefung. Die Umfassungswand ist in ein vorhandenes Bauteil integriert. Das verkörpert sich der Gestalt, dass die Umfassungswand der Dosierkammer aus elastischem Material besteht und einstückig mit dem Boden der Substanzen-Vorratskammer ausgebildet ist.

Weiter besteht noch eine bauliche günstige Maßnahme durch zwei beiderseits des zentralen Austragskanals in einer gemeinsamen Ebene zu diesem verlaufende Lufteinströmkanäle für die Substanzen-Vorratskammer. Das eröffnet die Möglichkeit einer schlanken Bauform, vor allem, wenn weiter die Lufteinströmkanäle in der Mittelebene zwischen taillenförmigen Ablageflächen-Einbuchtungen liegen.

Weiterbildend für einen handbetätigbaren Inhalator für pulverförmige Substanzen, insbesondere medikamentöse Substanzen, bei welchem sich bei der Handbetätigung eine bestimmte Ausgabemenge aus einer Substanzen-Vorratsmenge in einer Dosierkammer vor einem Austragskanal abteilt zwecks luftgetragener Ausgabe aus einer Mundstücköffnung am Ende des Austragskanals, wobei ein den Ausgabe-Luftstrom erzeugender Kolben mit einem Hohlraum des Schaftes eine Substanzen-Vorratskammer und die Dosierkammer bildet, wobei ein beim Rückhub des Kolbens erzeugter Unterdruck die Dosierkammer zur Substanzen-Vorratsmenge hin öffnet, und wobei weiter der Boden der Dosierkammer von einer luftdurchlässigen Membran gebildet ist, wird vorgeschlagen, dass die Dosierkammer in Grundstellung des Schaftes zur Substanzen-Vorratskammer hin geöffnet ist. Das lässt den Befüllungsweg offen, so dass ein hubunabhängiger, zeitlich gedehnter Eintrag für die Abgabemenge erreicht wird. Erst bei der Ausgabebetätigung, also in der Gebrauchsphase des Spenders, schließt sich die Dosierkammer. So bleibt in Grundstellung der Kontakt zwischen der in der noch offenen Dosierkammer bereitgehaltenen Ausgabemenge und der sich in der Substanzen-Vorratskammer befindlichen Vorratsmenge erhalten. Für in dieser Hinsicht kritische Substanzen kann das von Vorteil sein. Das Offenhalten ist mit einfachen, sogar bordeigenen Mitteln erreichbar, indem sich die Dosierkammer zur Substanzen-Vorratskammer hin durch einen Leerhub zwischen dem Schaft und der die Dosierkammer formenden Kolbenmanschette öffnet. Der Kolben fungiert bezüglich des Öffnens als Schleppkolben. Die Dosierkammer wird im Wechsel von Schlepp in Schub geöffnet bzw. geschlossen. Geber ist dabei der Reibschluss zwischen Kolbenlippe und Zylinderwand.

Vorteilhaft ist es überdies, dass der Boden der Substanzen-Vorratskammer Teil der Kolbenmanschette ist und an der Innenwand des Schachtes gleitend anliegt. Der Boden kann eine leichte diesbezügliche Vorspannung aufweisen, so dass zum einen dem Erfordernis der Dichtigkeit Rechnung getragen ist und zum anderen dem der guten Beweglichkeit.

Weiter ist so vorgegangen, dass die Dosierkammer teilweise von einer Vertiefung der Kolbenmanschette gebildet ist und teilweise aus einem sich dichtend auf den Rand der Vertiefung der Dosierkammer aufsetzenden, in Ausgaberichtung verjüngenden Ventilkegel besteht. Letzterer fungiert als feststehender Deckel, welchem die bewegliche Vertiefung genauer der Boden zugeht. Der Leerhub ist federveranlasst und dadurch gebildet, dass ein Kragen der Kolbenmanschette in einen entsprechend axial orientiert breiten Schlitz an der Innenwand des Schaftes ragt. Über den Öffnungshub wird der Dosierkammerspalt definiert. Bei dieser Ausgestaltung ist die Zylinderwand für die Kolbenmanschette von einem Stutzen einer Bodenplatte gebildet.

Dabei erweist es sich im Hinblick auf die Schaffung einer Federkammer als vorteilhaft, dass eine Kolbenfeder sich außenseitig des Stutzens erstreckt, und zwar in einem Ringspalt zwischen Stutzen und Außenwand des Inhalators.

Was den Ventilkegel angeht, so ist dieser bspw. auch kuppelförmig oder pyramidal gestaltet, und zwar weiter der Gestalt, dass der Ventilkegel, freigeschnittene, in Ausgaberichtung weisende Lippen besitzt, deren Trennschnittenden verdickt sind. Das erhält die ventillappentypische Beweglichkeit sowie Rückstellkraft und bringt trotzdem eine gut abgestützte, relativ großflächige Schließanlage an den korrespondierenden Rändern.

Der Gegenstand der Erfindung ist nachstehend anhand dreier zeichnerisch veranschaulichter Ausführungsbeispiele näher erläutert. Es zeigt:

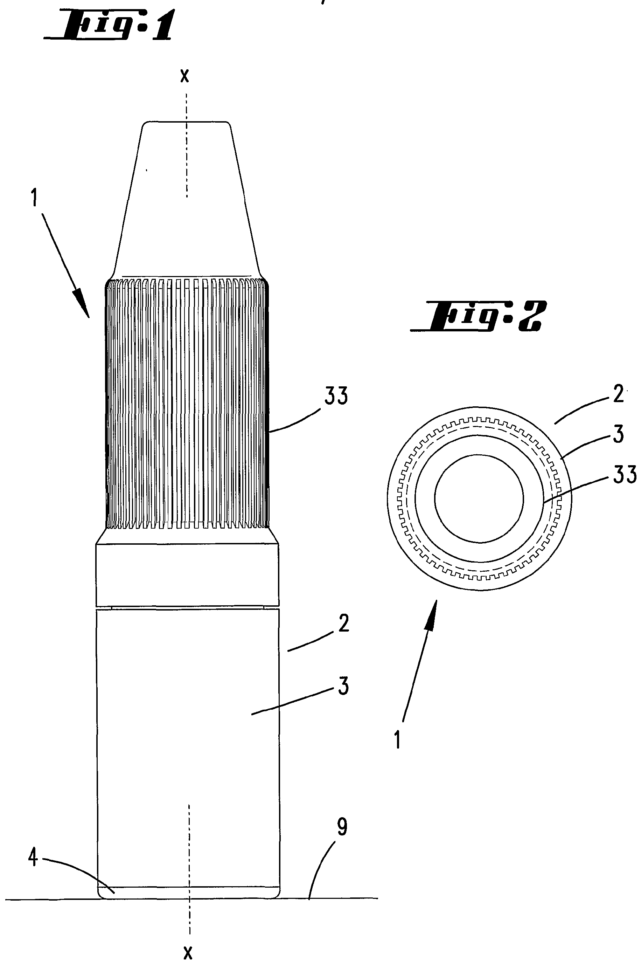

- Fig. 1: einen handbetätigbaren Inhalator in Seitenansicht, schutzkappenverschlossen, gemäß erstem Ausführungsbeispiel, vergrößert,
- Fig. 2: die Draufsicht hierzu,
- Fig. 3: einen Längsschnitt durch den kappenverschlossenen Inhalator, die federbelastete Grundstellung seines Kolbens wiedergebend,
- Fig. 4: den Inhalator in Betätigungsstellung, gleichfalls im Längsschnitt,
- Fig. 5a: auszugsweise eine Zwischenstellung unmittelbar nach abwärts ausgefedertem Boden der Substanzen-Vorratskammer, d.h. soeben geöffneter Dosierkammer, darstellend die (theoretische) momentane Volumenvergrößerung der Dosierkammer,
- Fig. 5b: eine später liegende Zwischenstellung bei schon wieder gefüllter Dosierkammer,
- Fig. 6: ein Detail der Abstützung der Decke des Inhalators,
- Fig. 7: einen Längsschnitt durch einen kappenverschlossenen Inhalator, die federbelastete Grundstellung seines Kolbens wiedergebend, gemäß zweitem Ausführungsbeispiel, vergrößert,
- Fig. 8: den Inhalator in Betätigungsstellung, gleichfalls im Längsschnitt,
- Fig. 9: den Querschnitt gemäß Linie IX-IX in Fig. 8,
- Fig. 10: einen Längsschnitt durch einen kappenverschlossenen Inhalator, die federbelastete Grundstellung seines Kolbens wiedergebend, gemäß drittem Ausführungsbeispiel, vergrößert,
- Fig. 11: den Inhalator in Betätigungsstellung, und zwar in der Anfangsphase, gleichfalls im Schnitt,

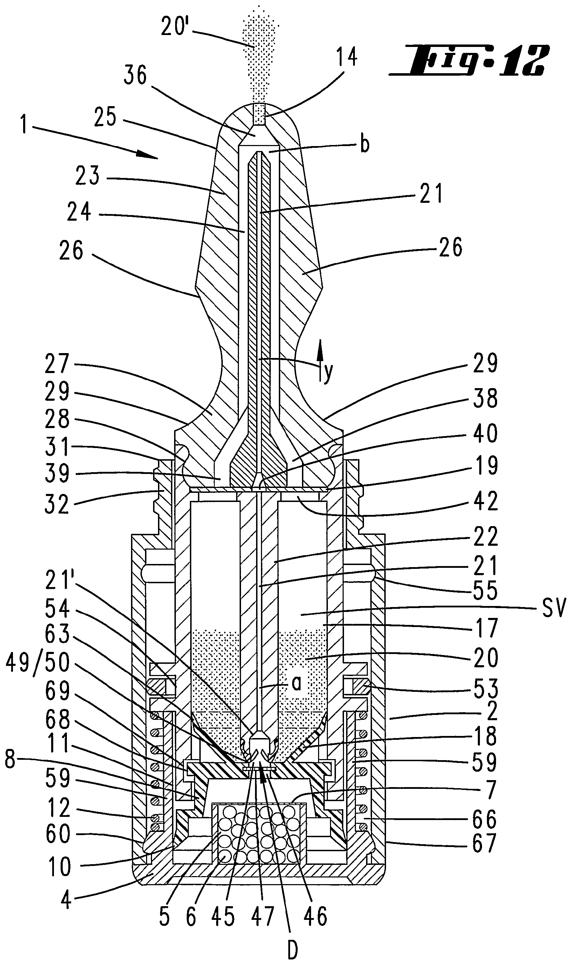

- Fig. 12: den Inhalator in Betätigungsstellung, und zwar nun in der Endphase befindlich, gleichfalls im Längsschnitt,
- Fig. 13: eine Herausvergrößerung des Bereichs der Dosierkammer, und zwar in Position gemäß Fig. 10, die Grundstellung wiedergebend,
- Fig. 14: das Ventil in isolierter Wiedergabe,

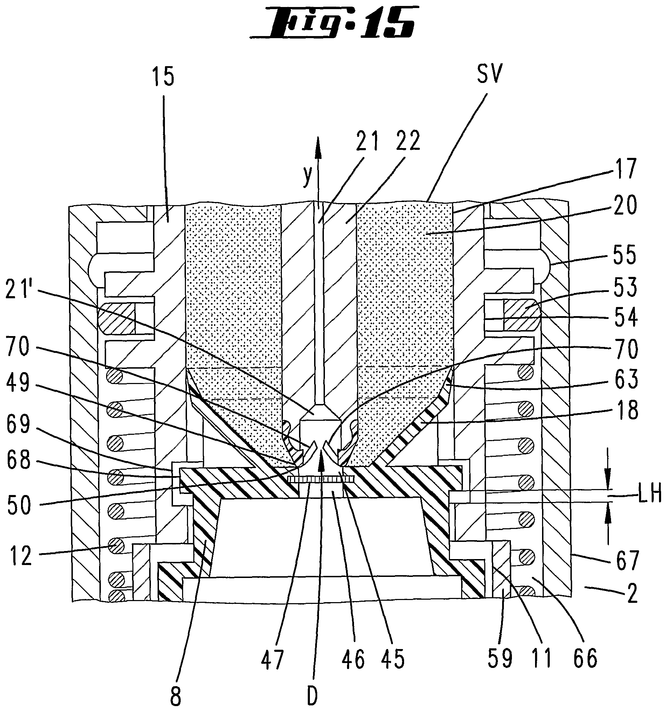

- Fig. 15: den Bereich der Dosierkammer in Herausvergrößerung bei zur Ausbringung geöffnetem Ventil,
- Fig. 16: das Ventil in perspektivischer Darstellung, ausgebildet als Ventilkegel,
- Fig. 17: eine Darstellung wie Fig. 16 in pyramidaler Ausgestaltung des Ventils,
- Fig. 18: ebenfalls in schaubildlicher Darstellung die entsprechende Pyramidenfigur allein.

Der dargestellte, als Taschengerät ausgebildete Inhalator 1 besitzt ein im Grunde kreisrunden Querschnitt aufweisendes Gehäuse 2. Bestandteil desselben ist ein Zylinder 3 als Teil einer Kolben/Zylinder-Einheit, fungierend als Pumpe.

Der Zylinder 3 ist basisseitig durch eine Bodenkappe 4 dicht verschlossen. Die Bodenkappe 4 ist in den dortigen Endbereich des Zylinders 3 eingeklipst.

Die Bodenplatte 4 stellt eine Trockenmittelkammer 5. Die diesbezügliche Substanz ist durch Kügelchen 6 dargestellt. Überdeckt wird die Trockenmittelkammer 5 von einer im Zylinder 3 gehalterten Lochplatte 7 oder aufgesiegelten perforierten Folie.

Im Zylinder 3 führt sich ein längsverlagerbarer Kolben 8. Der ist als Kolbenmanschette realisiert. Seine in Richtung einer der Standfläche 9 weisende, leicht ausgestellt Kolbenlippe 10 führt sich abgedichtet, mit leichter Vorspannung an der Zylinderwand 11 des Zylinders 3. Der Kolbenhub ist endanschlag-definiert. Die Ausrichtung der Kolbenlippe 10 führt dazu, dass bei Überschreiten eines bestimmten Unterdruckes die Lippe 10 von der Wand 11 abhebt, dies im Sinne einer Unterdruckbegrenzung durch die dann in den Raum unter dem Kolben 8 einströmende Luft.

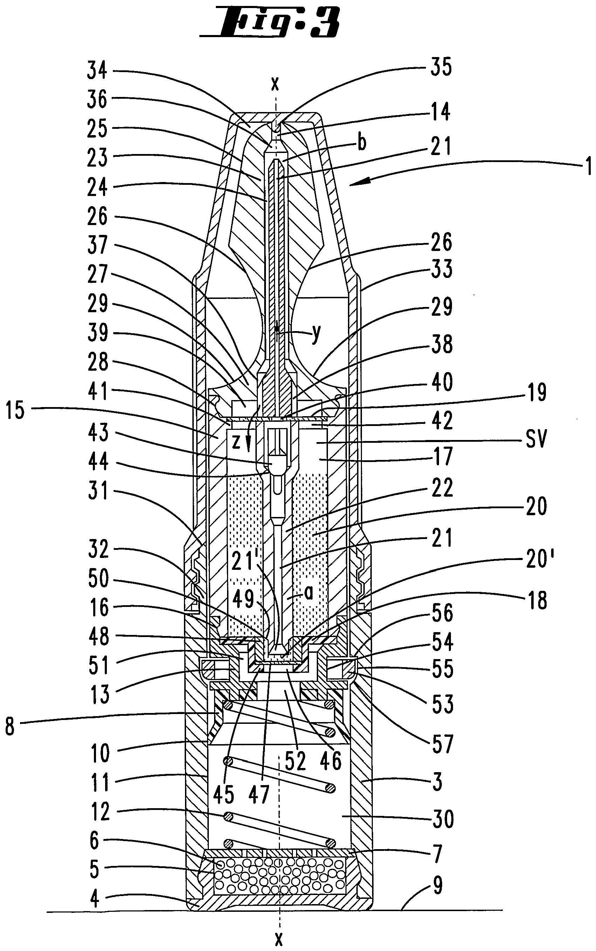

Der Kolben 8 steht unter Federbelastung im Sinne seiner Grundstellung (Fig. 3) des Inhalators 1. Die Feder, eine Schraubengangdruckfeder, trägt das Bezugszeichen 12.
Deren eine endständige Federwindung ragt in die Höhlung des Kolbens 8, also in die der Kolbenmanschette, die andere findet ihr Widerlager an der ortsfesten Lochplatte 7.

Die elastische Kolbenmanschette sitzt an einem Kolbenkopf 13 aus relativ härterem Material. Die Teile 8 und 13 können im Kombi-Spritzverfahren erstellt werden. Angewendet wird ein Mehrkomponenten-Spritzverfahren.

Der Kolbenkopf 13 setzt sich basisabgewandt in einen in Richtung einer Mundstücköffnung 14 verlaufenden Schaft 15 fort. Der ist hohl-zylindrischer Gestalt und mit dem Kolbenkopf 13 fest verbunden. Eine entsprechende Raststelle ist mit 16 bezeichnet. Rastwulst und Rastnut sind der Zeichnung entnehmbar.

Ein Hohlraum 17 des Schaftes 15 ist zur Bildung einer Substanzen-Vorratskammer SV herangezogen. Diese schließt kolbenseitig mit einem Boden 18 und mundstücköffnungsseitig einer Decke 19 ab.

Bezüglich der Substanz handelt es sich um pulverförmige, insbesondere medikamentöse Substanz, deren Vorratsmenge in der Zeichnung mit 20 bezeichnet ist. Von dieser Substanzen-Vorratsmenge 20 werden durch Handbetätigung des Inhalators 1 exakt reproduzierbare Ausgabemengen 20' in einer Dosierkammer D abgeteilt. Das Abteilen geschieht räumlich vor einem Austragskanal 21, und zwar am dem Kolben 8 zugewandten, unteren Ende a eines Schaft-Innenrohres 22. Das Schaft-Innenrohr 22 befindet sich im Zentrum des Schaftes 15 des unter Federspannung stehenden Kolbens 8. Das Schaft-Innenrohr 22 passiert bzw. durchsetzt dabei nicht nur den gesamten Längenbereich des Hohlraumes 17, sondern setzt sich auch noch in weiteres, das Schaft-Innenrohr 22 umschließendes Schaftmaterial 23 fort.

Die geometrische Längsmittelachse des zentralen Schaft-Innenrohres 22 fällt mit einer rotationssymmetrisch liegenden Längsmittelachse x-x des Inhalators lzusammen. Insofern liegt die Vorratsmenge 20 in einem Ringraum, aus welchem austretend die Ausgabemenge 20' unter dem kolbenseitigen Ende a des Schaft-Innenrohres 22 gesammelt wird, dies zwecks späterer Ausgabe aus der Mundstücköffnung 14 am anderen, also oberen Ende b des Austragskanals 21.

Das Schaft-Innenrohr 22 erstreckt sich bis kurz vor die Mündungsöffnung 14. Der Mantelbereich des Schaft-Innenrohres 22 wird unter Belassung des radialen Abstandes vom Schaftmaterial 23 umgeben, genauer dessen Wand, so dass über die gesamte Länge des Schaftmaterials 23 ein Ringraum verbleibt. Der stellt einen konzentrisch zum Austragskanal 21 angeordneten Lufteinströmkanal 24. Letzterer reicht bis in die Substanzen-Vorratskammer SV und steht via Mundstücköffnung 14 im Anschluss zur Atmosphäre. 14 ist gleichsam auch ein Atemloch, insbesondere auch zum Ausgleich des sich verringernden Volumens an Pulver.

Bezüglich des Schaftmateriales 23 handelt es sich um einen keulenförmigen Fortsatz 25 des Schaftes 15. Dessen freies Ende konvergiert kegelstumpfförmig unter Bildung einer Kopfrundung im Bereich der Mundstücköffnung 14. Ein solcher Stutzen lässt sich gut geführt in beispielsweise ein Nasenloch des Benutzers einstecken.

Basisseitig des Fortsatzes 25 liegen Einbuchtungen 26. Die gehen, Grund gegen Grund gerichtet, nach einer konkaven Einziehung in einen breiten Sockel 27 über. Zwischen dem Sockel 27 und dem dortigen Ende des Schaftes 15 ist wiederum eine Raststelle berücksichtigt, bezeichnet mit 28. Auch diese weist einen Rastwulst und eine passende Rastnut auf, wie sich das aus der Zeichnung ergibt.

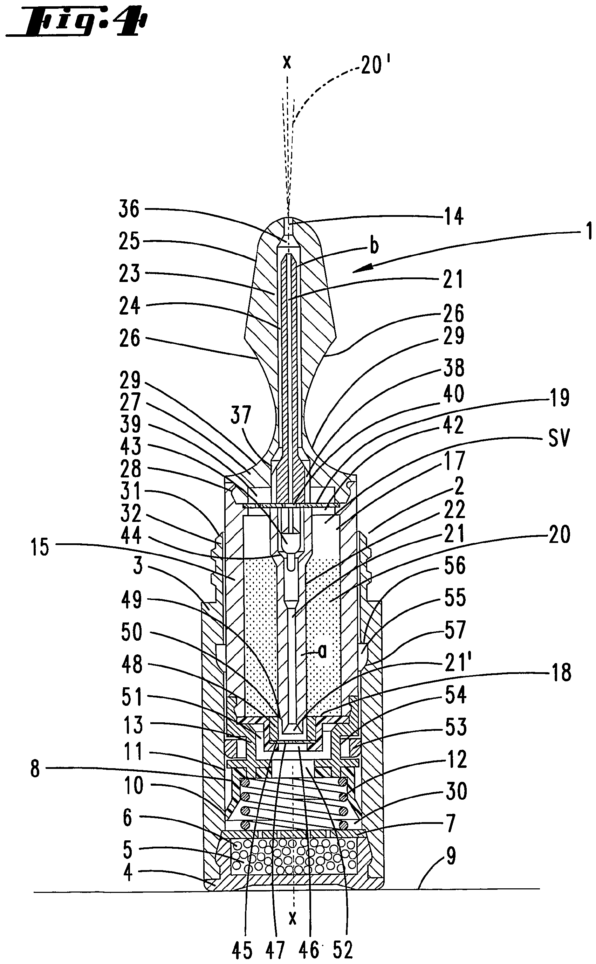

Der schulterbildende Abschnitt des keulenförmigen Fortsatzes 25 lässt aufgrund der Einbuchtungen 26 am Sockel 27 Fingerauflageflächen 29 entstehen, über welche sich der Kolben 8 via Schaft 15 entgegen Federbelastung in die Stellung gemäß Fig. 4 verlagern lässt.

Der Zylinderraum 30 des Kolbens 8 weist eine axiale Länge auf, die etwa dem Kolbendurchmesser entspricht. Mindestens um dieses Maß ragt der mundstücköffnungsseitige Endabschnitt des Schaftes 15 über einen Halsrand 31 des Zylinders 3 axial vor. Der Halsrand 31 ist der obere Abschluss eines Halses 32 des Zylinders 3. Die Mantelfläche des Halses 32 trägt ein Außengewinde, welches mit einem passenden Innengewinde eines Schraubsockels der Schutzkappe 33 des Inhalators 1 zusammenwirkt. Die Mantelfläche der Schutzkappe 33 kann gerauht, insbesondere längsgerieft sein, dies zur Erleichterung der Schraubbetätigung.

Von einem ausgabeseitig konvergierenden Dom 34 der besagten Schutzkappe 33 geht innenseitig einer abgeflachten Decke derselben ein Verschlussstopfen 35 aus. Der tritt bei ordnungsgemäß geschlossenem Gerät dichtschließend in die Mundstücköffnung 14 ein.

Unterhalb der besagten Mundstücköffnung 14 ist zwischen dem dortigen Ausgang des Austragskanals 21 und dem Ansatz der Mundstücköffnung 14 eine Zwischenkammer 36 belassen, bildend eine gegenläufige Strömungsweiche für den Lufteinströmkanal 24 einerseits und den Austragskanal 21 andererseits. Das Ende des Schaft-Innenrohres 22 ist zugespitzt. So entsteht eine schräge Leitschulter für die Einströmluft.

In der Ebene des Sockels 27 nimmt der Lufteinströmkanal 24 eine leichte Ausstellung ein. Das Schaft-Innenrohr 22 ist dort wandungsmäßig leicht verdickt. Der verdickte Bereich sitzt in einer passenden Ausnehmung 37 im Sockel 27. Es liegt ein reibungsschlüssiger Steckverbund vor, wobei kanalbildend zwei oder mehrere längsverlaufende Nuten 38 ausgebildet sind. Die Nuten 38 leiten zu einem Freiraum 39 oberhalb der Decke 19.

Auf Höhe der Decke 19 ist der Körper des Schaft-Innenrohres 22 unterbrochen, strömungsmäßig jedoch nicht. Das prägt sich dadurch aus, dass im oberen Bereich der Substanzen-Vorratskammer SV eine für die Einströmluft durchlässige Decke 19 ausgebildet ist, die das Schaft-Innenrohr 22 beiderseits abstützend kreuzt. Über die vertikal querende Fortsetzung des Austragskanals 21 weist die Decke 19 dagegen ein Loch 40 auf. Die entsprechende Durchlässigkeit bietet beispielsweise ein Filterpapier.

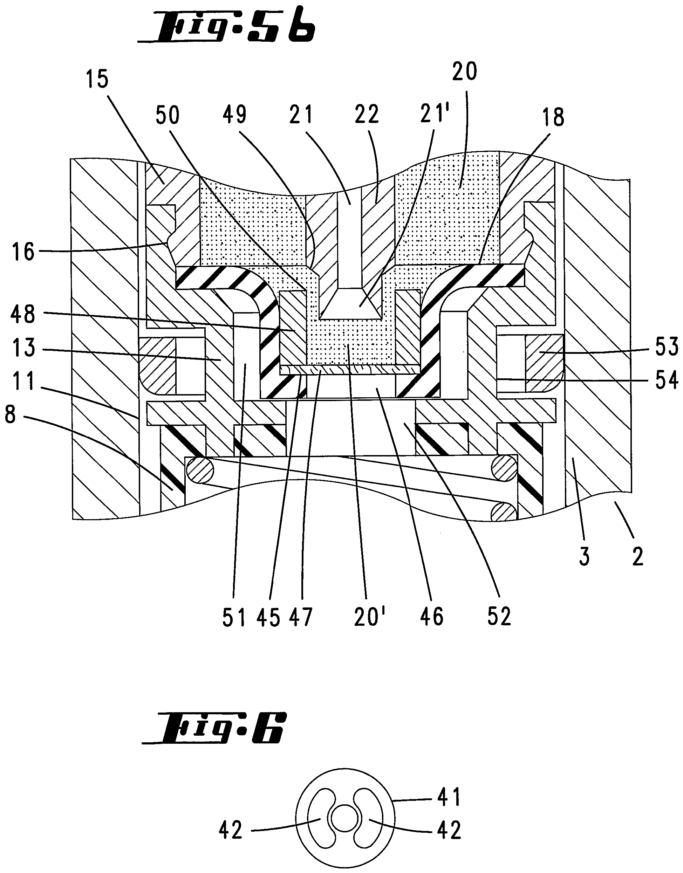

Die Decke 19 ist abgestützt von einer durchbrochenen Halterung 41, welche Durchströmöffnungen 42 belässt. Es kann sich um stegbeabstandete Bogenöffnungen handeln (vergl. Fig. 6). Das schaftnahe Stegmaterial bildet eine sichere Auflage für die Decke 19, die oberseitig durch einen Ringkragen der Raststelle 28 gegen die Halterung 41 gehend randeingeklemmt ist.

Eine gleiche Klemmhalterung ist auch lochnah realisiert. Durch Gegeneinandertritt der verbreiterten Enden des zweiteiligen Schaft-Innenrohres 22 im Querungsbereich der Decke 19. Der im Hohlraum 17 aufgenommen Part des Innenrohres 22 ist über die Halterung 41 einteilig mit dem Schaft 15.

Vor dem zentralen Loch 40 befindet sich ein Ventilkörper 43. Der wirkt mit einer Ventilsitzfläche 44 zusammen. Ein solches Rückschlagventil wirkt in Austragsrichtung öffnend, schließt dagegen bei Einströmen der Luft über den Kanal 21. Das Rückschlagventil wird vom substanztragenden Strom umspült. Die zugehörige Ventilkammer ist durch entsprechende Ausweitung des dortigen Rohrendes erreicht. Der Ventilschaft des Ventilkörpers 43 weist kreuzende Flügel auf, die aber im Kern das Loch 40 bei Luftaustrag nicht zuhalten können.

Die von der Substanzen-Vorratsmenge 20 abzuteilende Ausgabemenge 20' sammelt sich in einer Dosierkammer-Vertiefung 45 im Boden 18 der Substanzen-Vorratskammer SV. Es handelt sich um einen hutförmig oder topfförmig gestalteten Körper aus elastisch flexiblen Material. Der Hutrand ist im Bereich der Raststelle 16, ähnlich wie oben bezüglich der Decke 19 ausgeführt, randeingeklemmt.

Der Boden 18, genauer die Decke des umgekehrt hutförmig gestalteten bzw. eingesetzten Körpers weist zentral liegend ein Loch 46. Dieses Loch 46 besitzt eine luftdurchlässige Membran bzw. Abdeckung 47. Die Struktur des entsprechenden Filtermaterials ist so, dass die pulverförmige Substanz in beiden Fällen nicht passieren kann, vielmehr - wie schon gesagt - nur Luft, und dies auf jeden Fall in Richtung der Mundstücköffnung.

Die Topfinnenwand des topf- bzw. hutförmigen Körpers trägt ein hülsenförmiges Einsatzteil 48, gleichsam als Aussteifung an der Topfinnenwand wirkend. Auf dessen oberem inneren Rand sitzt das Ende a des Schaft-Innenrohres 22 in Grundstellung dichtend auf. Dies geschieht aufgrund der dem Boden 18 innewohnenden Rückstellkraft. Der besagte Rand trägt das Bezugszeichen 50. Die endseitige Gegenschließfläche am ortsfesten Innenrohr 22 ist mit 49 bezeichnet. Letztere ist als Kegelstumpfzone verwirklicht, die sich in ein im Außendurchmesser reduziertes Endstück fortsetzt, das in Gegenrichtung getrichtert ausgebildet ist. Das reduzierte Endstück taucht in den Topfabschnitt mit ein. Das Endstück stellt die Mündung zum Austragskanal 21 dar. Das optimiert den Austrag der Substanz. Der Trichter 21' verjüngt sich in Mundstückrichtung. Das ergibt eine kelchartige Komprimierung für die luftgetragene pulverförmige Substanz, die sich dadurch im engeren Kanalabschnitt strömungsbeschleunigt. Es entsteht ein kraftvoller Strahl, der das Medikament auch in Nebenhöhlen transportiert. Andererseits findet aber auch kein als unangenehm empfundener Strömungsschlag statt. Dazu ist die Luftmenge begrenzt. Als vorteilhaft hat sich hier erwiesen, dass das aus der Kolbenbewegung resultierende Luftstrom-Volumen mehr als das Hundertfache des Volumens der Dosierkammer D beträgt, aber weniger als das Sechshundertfache. So sind auf 4 ml Luftstrom ca. 10 mg pulverförmige Substanz verteilt. Das Volumen der Dosierkammer liegt bei 10 mm³. Hinsichtlich der Porenweite der luftdurchlässigen Membran bzw. Abdeckung 47 ist auf ca. 1 µ gesetzt. Eine solche Formel ist auch bezüglich der mechanischen Belastbarkeit der Spendermechanik günstig, so dass die Lebensdauer des Gerätes zufriedenstellend ist.

Die Vertiefung 45, d. h. das sie umschreibende Topfteil des Bodens 18, ragt mit dem bewegungsnotwendigen Freistand in eine Ausnehmung 51 des Kolbenkopfes 13. Auch hier liegt eine Topfform zugrunde mit radialem und axialem Ausweichspiel für den Boden 18. Kolbenkopf 13 und Kolben 8 sind zentral durchbrochen. Die entsprechende Öffnung trägt das Bezugszeichen 52. Sie nimmt strömungsmäßig Anschluss an den Zylinderraum 30 der Pumpe, der so mit der Ausnehmung 51 verbunden ist.

Dem Inhalator 1 ist im Hinblick auf die Verlagerung des Kolbens 8 unter Nutzung des Fortsatzes 25 als Betätigungshandhabe eine Ansprechschwelle für die handbetätigte Kolbenverlagerung gegeben. Bestandteil dieser bei Überlast nachgebenden Anfangsabstützung ist ein federnder Ringkörper 53. Der ist einerseits mit dem Kolbenschaft 15 verbunden. Er sitzt dazu in einer Ringnut auf dem halsartigen Nacken 54 des Kolbenkopfes 13. Besagter Ringkörper 53 ist so daran axial gefesselt. Er ragt mit zwei diametral einander gegenüberliegenden Vorsprungszonen in eine Rastnut 55. Die befindet sich in der Zylinderwand 11. Die Rastnut 55 weist eine obere, steile Flanke 56 auf und eine untere, einwärts gerichtet abfallende Flanke 57. Letztere lenkt die die Ansprechschwelle bildenden bügelzungenartigen Vorsprungszonen schlagartig ein, so dass der Gegenhalt plötzlich zusammenbricht. Es kommt zu einem schlagartigen Verlagern des Kolbens 8 unter Komprimierung der im Zylinderraum 30 befindlichen Luft. Der Öffnungsdruck auf die Feder 12 nebst Ringkörpers 53 liegt bei ca. 2,5 kg. Das ist ein willensbetont aufbringbarer Wert im ergonomisch bestens beherrschbaren Bereich. Wieder freigegeben, schnäppert der Ringkörper 53 erneut sperrend in die Rastnut 55 zurück. Das ist die anschlagbegrenzte Grundstellung; der Ringkörper 53 liegt an der steileren Flanke 57 an.

Die Funktion des Inhalators 1 ist wie folgt: Durch Positionieren der Finger der Bedienungshand auf den Fingerauflageflächen 29 und einen dem Spanngriff gemäßen Gegenhalt der Bodenplatte 4 durch den Daumen lässt sich in solchem Spanngriff der Kolben 8 in der geschilderten Weise schlagartig entgegen Federbelastung abwärts bewegen. Die sich im volumenmäßig verringernden Zylinderraum 30 befindliche, komprimierende Luft schlägt durch die luftdurchlässige Membran bzw. Abdeckung 47 in die gegenüber der Vorratsmenge 20 versperrte Dosierkammer D, in welcher die Ausgabemenge 20' aus einer voraufgegangenen Betätigung bereitgehalten ist. Es kommt zu einem kraftvollen, gleichwohl gedämpften Ausstoß der luftstromgetragenen pulverförmigen Substanz an den Zielort, bspw. über den Nasenraum. Der Ventilkörper 43 hebt dabei von der Ventilsitzfläche 44 ab. Zwischen der Fläche 49 des Schaft-Innenrohres 22 und dem korrespondierenden Rand 50 herrscht Dichtschließung. Die Ausgabe der abgeteilten Menge 20' ist lagenunabhängig.

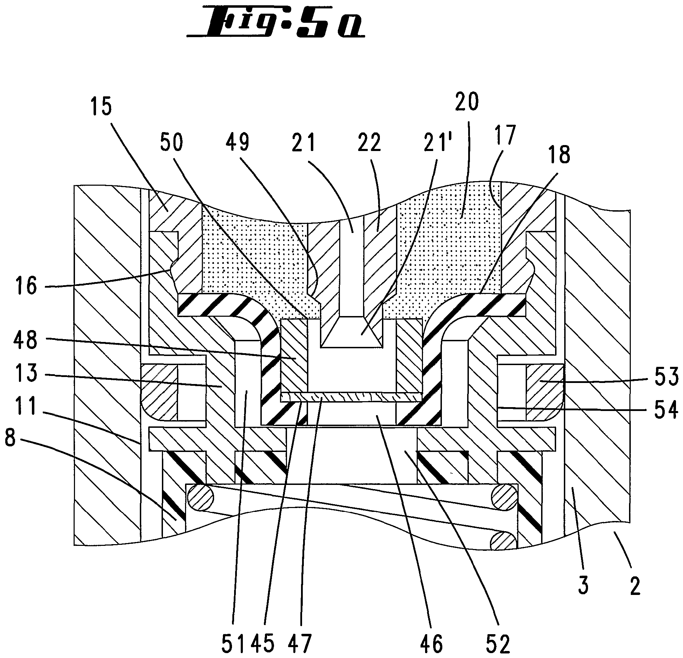

Gibt der Benutzer die Betätigungsstellung des Inhalators 1 frei, bewegt sich vermöge der Federkraft der Feder 12 der Kolben 8 wieder in seine Grundstellung. Dabei kommt es zur Volumenvergrößerung der Pumpenkammer, also des Zylinderraumes 30. Der hutförmige Körper, der Boden 18, wird durch den nun herrschenden Unterdruck entgegen seiner elastischen Rückstellkraft abwärts gezogen in die Stellung nach Fig. 5a: Die (leere) abteilende Dosierkammer D ist geöffnet zur Substanzen-Vorratskammer SV hin. Es kommt zu einem erneuten Befüllen der Dosierkammer D, d.h. eine genau dosierte Ausgabemenge 20' wandert aus der Vorratsmenge 20 heraus in die Dosierkammer D, dies teils durch Eigengewicht, teils durch Massenträgheit, teils durch die Vergrößerung des Kammervolumens zufolge Abwärtsziehens des topfförmigen Körpers, und, je nach Bemaßung der Luftdurchlässigkeit, der Membran respektive Abdeckung 47 in Abwärtsrichtung, eben begünstigt durch eine Luftströmung in Richtung der Abdeckung bzw. der Membran 47, vor allem aufgrund der Volumenvergrößerung durch diese Abwärtsbewegung. Die erste, recht dünne Schicht des Pulvers schließt die Poren der Abdeckung 47, so dass die Öffnungsstellung zunächst erhalten bleibt. Der Weg der elastischen Verlagerung des Bodens 18 in die Öffnungsstellung der Substanzen-Vorratskammer SV ist begrenzt dadurch, dass beim Rückhub des Kolbens 8 der hinter, genauer unter diesem auftretenden Unterdruck begrenzt ist durch die abwärts gerichtete Lippe 10 des Kolbens 8, die sich bei zu großem Unterdruck perfekt ausgleichend von der Wand 11 abhebt, um Luft in den Raum 30 zu lassen. Der Saugstrom hebt den Rand 50 von der Gegenfläche 49 des Endes a des Schaft-Innenrohres 22 so lange ab, wie der Unterdruck existiert. Es findet ein einwandfreies Anfüllen statt (Fig. 5b). Die pulverförmige Substanz wird gegenüber dem Zylinderraum 30 durch die luftdurchlässige Membran bzw. Abdeckung 47 zurückgehalten. Es findet kein Verfälschen der Ausgabemenge 20' statt, insbesondere weil die Substanz stets locker bleibt. Ist der Kolben 8 wieder in der Hochstellung (Fig. 3), tritt der Boden 18 aufgrund seiner elastischen Rückstellung wieder in die Schließstellung nach Fig. 3, welche Aufwärtsbewegung auch noch eine gleichmäßige satte Füllung der Dosierkammer D begünstigt. Der jeweils zwischen einer Dicht- und Öffnungsstellung des Schaft-Innenrohres 22 wechselnde Rand 50 gelangt stets sicher in die die abteilende Dosierkammer D schließende Dichtstellung, zumal ein etwaiger Überdruck im Kanal 21 entweichen kann.

Die Austragsströmung ist mit Pfeil y und die Einlassströmung mit Pfeil z bezeichnet.

Die Raststelle 28 kann als Füllzugang öffenbar ausgebildet sein.

Der handbetätigbare Inhalator 1 gemäß zweitem Ausführungsbeispiel entspricht prinzipiell und weitestgehend auch baulich der eingehend geschilderten Grundversion, welche das erstes Ausführungsbeispiel verkörpert. Die Bezugssymbole sind, soweit zum Verständnis erforderlich, sinngemäß angewandt, zum Teil ohne textliche Wiederholungen, da der entsprechende Offenbarungsinhalt auf die Grundversion lesbar.

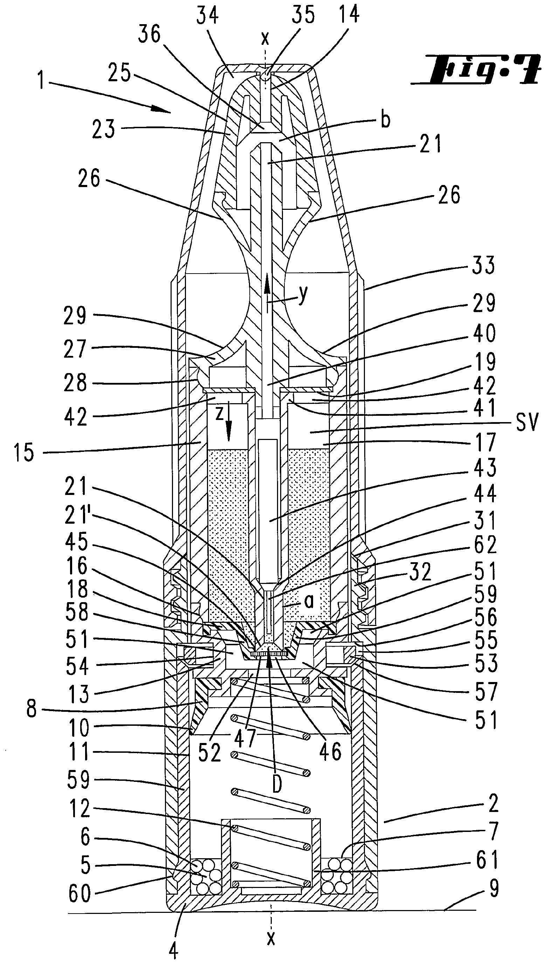

Ab Fig. 7ff. ist eine Modifikation der Dosierkammer D vorgenommen. Statt die Gegenschließfläche 49 an einem axial zurückliegenden Abschnitt des unteren Endes a des Schaft-Innenrohres 22 auszubilden, ist diese nun direkt von der gehöhlten Stirnfläche des besagten Schaft-Innenrohres 22 gestellt. Das verkörpert sich lösungsmäßig im Einzelnen darin, dass die Dosierkammer D nun von einem endseitigen Erbreiterungsbereich des auf die luftdurchlässige Membran oder Abdeckung 47 direkt aufsitzenden Austragskanals 21 gebildet ist. Der so stumpf aufsitzende Austragskanal 21 hebt beim Federhub der Feder 12 wie geschildert von dem ortsfesten deckelartigen, gleichsam austulpenden Erbreiterungsbereich ab. Geometrisch handelt es sich bei diesem Erbreiterungsbereich auch hier um einen Trichter 21'. Selbstredend entfernt sich der auch hier elastische rückstellfähige Boden 18, welchem die luftdurchlässige Membran bzw. Abdeckung 47 eingeformt ist, nach unten gehend und hebt ab. Dazu ist das Umfeld des auch hier topfförmigen Körpers von einer axial wie radial nach auswärts freigebenden Ausnehmung 51 versehen.

Die Peripherie des Bodens 18 weist eine vertikale rotationssymmetrische Abwinklung auf. Die ist im Bereich der Raststelle 16 zwischen dem Schaft 15 und dem auch hier vorgesehenen Kolbenkopf 13 sicher gefesselt.

Eine Umfassungswand 58 des Bodens 18, umschreibend die Dosierkammer D, bildend die genannte topfförmige Ausprägung ist hier konkret kegelstumpfförmig. Diese einem Blumenpflanztopf vergleichbare Struktur ist so, dass die Umfassungswand 58 der Dosierkammer D sich in Austragsrichtung erweitert, kenntlich gemacht durch Pfeil y betreffend die Austragsströmung.

Auch die Umfassungswand 58 der Dosierkammer D besteht aus elastischem Material und ist einstückig mit dem Boden 18 der Substanzen-Vorratskammer SV ausgebildet.

Die kegelstumpfförmige Umfassungswand 58 hält befüllfähigen Abstand zur korrespondierenden Mantelwand des Endes a des Schaft-Innenrohres 22. Die rotationssymmetrisch nach hinten hin gleichsam ausspitzende Ringzone stellt eine vorteilhafte Sammelrinne unmittelbar am Fuß der trichterförmigen Decke der Dosierkammer D.

Der ringrippenartig auskeilende, bezüglich der luftdurchlässigen Membran bzw. Abdeckung 47 aufsetzende Rand des Trichters 21' weist einen Durchmesser auf, der im Wesentlichen dem des Loches 46 der Dosierkammer-Vertiefung 45 entspricht.

Hinsichtlich des Volumens der Dosierkammer D und des die pulverförmige Substanz fördernden Luftstrom-Volumens liegen auch hier dieselben Parameter vor. In baulicher Hinsicht bleibt noch festzuhalten, dass die Zylinderwand 11 nun nicht mehr von dem bereits das Gehäuse 2 mitbildenden Zylinder 3 gestellt ist, sondern von der Bodenplatte 4. Der entspringt ein Stutzen 59. Der Stutzen 59 ist rastgesichert in das Gehäuse 2 von unten her eingesteckt. Er reicht unter Belassung eines axialen Abstandes vor der in den Hals 32 übergehenden Schulter des Gehäuses 2. Die die Ineinanderschachtelung sichernde Raststelle trägt das Bezugszeichen 60. Sie umfasst den üblichen Rastwulst sowie eine passende Rastnut.

Der besagte axiale Abstand zur besagten Schulter ist nunmehr im Sinne der Schaffung einer Rastnut 55 für den Ringkörper 53 genutzt, auch hier stellend die steile Flanke 56 und die abfallende Flanke 57. Letztere ist das trichterartig gestaltete Stirnende des Stutzens 59.

Zentral gelegen wird die Bodenkappe 4 zur Ausbildung einer Federkammer für die Feder 12 herangezogen, welche im vorliegenden Fall ebenfalls als Schraubengangdruckfeder realisiert ist. Kammerbildend ist dabei eine vom Kappenboden 4 ausgehende Ringwand 61. Die dosierseitige Stirnfläche der Ringwand 61 fungiert als Begrenzungsanschlag für den Kolbenkopf 13. Es tritt Hartteil gegen Hartteil.

Beim Gegenstand gemäß zweitem Ausführungsbeispiel ist die Trockenmittelkammer 5 nunmehr als Ringkammer gestaltet, aufnehmend die abgedeckten Kügelchen 6, nun in Form einer Ring-Lochplatte 7 oder -folie.

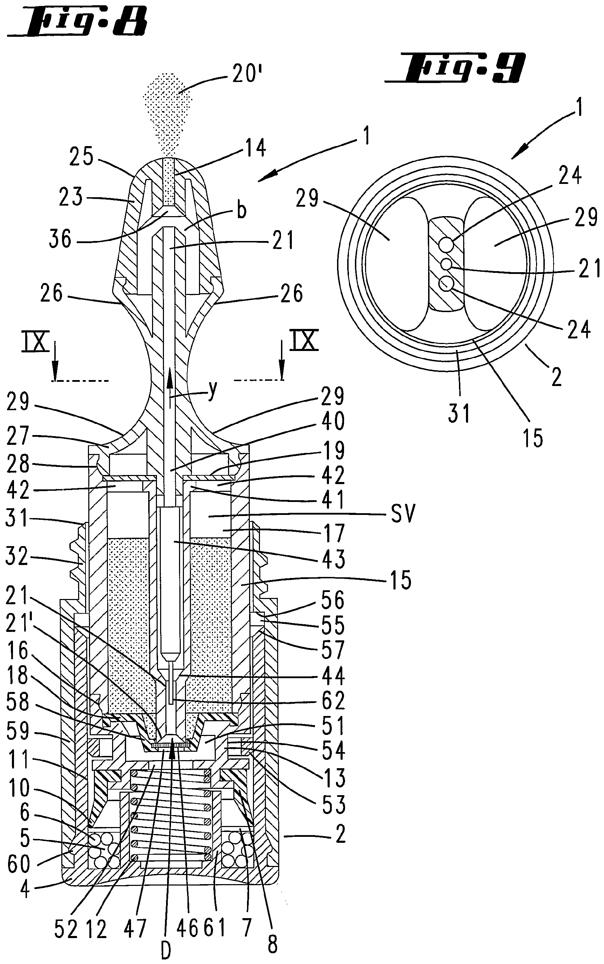

Was den Betätigungsbereich des Inhalators 1 angeht, so sind hier gleiche Strukturen angewandt. Ein Unterschied gegenüber der Grundversion besteht lediglich darin, dass nunmehr der zentrale Einströmkanal 24 ersetzt ist durch einen Doppelkanal als Luftführung für den Druckausgleich im Pulverreservoir sprich Vorratsmenge 20. Es sei auf Fig. 9 verwiesen. Dort ist entnehmbar, dass die beiden Lufteinströmkanäle, ebenfalls mit 24 bezeichnet, in der Mittelebene zwischen taillenförmigen bzw. -bildenden Auflageflächen-Einbuchtungen 26 liegen. Sie erstrecken sich beiderseits des zentralen Austragskanals 21 in einer gemeinsamen Ebene. Sie weisen einen größeren Querschnitt auf als der des zentralen Austragskanals 21 beträgt. Das Verhältnis liegt etwa bei 2:1. Solche Bohrungen in eine gemeinsame Ebene zu legen ermöglicht es, die Einbuchtungen 26 tiefer einschneidend auszubilden, so dass hieraus entsprechend großflächigere Fingerauflageflächen 29 entstehen.

Funktion und Arbeitsweise des Ventilkörpers 33 entsprechen der Grundversion, nur dass die Ventilsitzfläche 44 nunmehr eine größere Nähe zum Boden 18 der Substanzen-Vorratskammer SV einnimmt und der Ventilkörper 33 insgesamt länger gestaltet ist.

Bodenseitig setzt sich der Ventilkörper 43 überdies in einen Zentrierstift 62 fort. Der ragt in den querschnittskleineren Abschnitt des Schaft-Innenrohres 22.

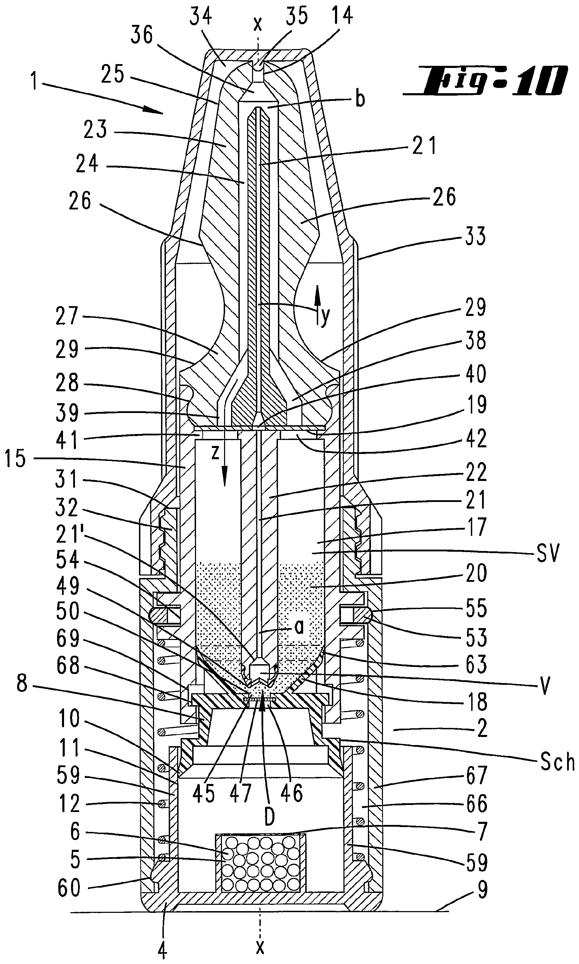

Was den handbetätigbaren Inhalator 1 gemäß drittem Ausführungsbeispiel betrifft (Fig. 10ff.), so liegt dessen funktionale und bauliche Ausprägung im geschilderten Rahmen der voraufgegangenen Ausführungsbeispiele. Auch hier sind die Bezugsziffern sinngemäß angewandt, dies zum Teil ohne textliche Wiederholungen, da auf die vorerörterten Lösungen lesbar.

Ein weiterbildender Unterschied besteht in der Art der Kolbenzuordnung zur Steuerung der Öffnungs- und Schließbewegung der Dosierkammer D. Im Gegensatz zur den geschilderten Vorläufern ist die Dosierkammer D in der federbelasteten Grundstellung des Schaftes 15 zur Substanzen-Vorratskammer SV hin geöffnet. Das lässt den Befüllweg in die Kammer D offen, so dass beim Hantieren, Aufstellen etc. des Inhalators 1 die pulverförmige Substanz bevorzugt schon schwerkraftmäßig sich ansammelt. Erst bei der Ausgabebetätigung schließt die Dosierkammer D, und zwar gleichsam streichmaßartig, indem der überschüssige Vorrat an pulverförmiger Substanz gleichsam weggedrängt wird. Hinzu kommt noch die vorteilhafte Wirkung einer Verdichtung. Das eröffnet exakt reproduzierbare Ausgabemengen 20' an pulverförmiger Substanz und zwar in Anwendung der Verhältnisse Pulverpartikel-Menge zum Luftstrom-Volumen, wobei überdies der Boden der Dosierkammer D von der luftdurchlässigen Membran bzw. Abdeckung 47 gebildet ist.

Zum entsprechenden Offenhalten der Dosierkammer D ist der Kolben 8 respektive die ganze Kolbenmanschette als Schleppkolben Sch realisiert.

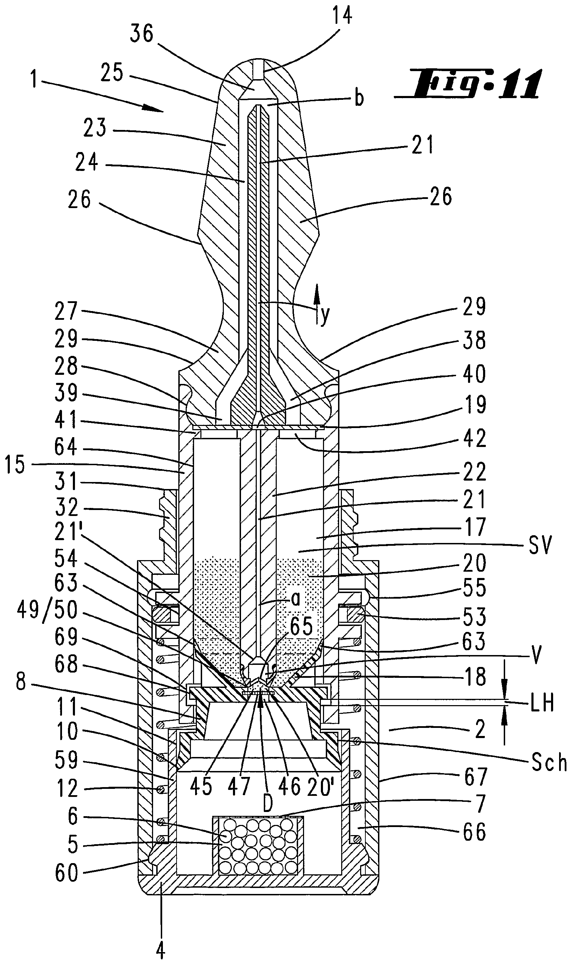

Das entsprechende Umschalten ergibt sich durch Wechsel von Schlepp in Schub, was zu einem Öffnen bzw. zu einem Schließen führt. Baulich ist das dadurch bewältigt, dass sich die Dosierkammer D zur Substanzen-Vorratskammer SV hin durch einen Leerhub LH zwischen dem Schaft 15 und der die Dosierkammer formenden Kolbenmanschette öffnet. Ein entsprechender Freigang zwischen den beiden kolbenbildenden Teilen ist Fig. 11 entnehmbar. Bei der hier zugrunde liegenden Baugröße des Inhalators 1 liegt der Öffnungshub bei 0,3 mm. Ein dementsprechender Abstand liegt so auch zwischen der ventilbewehrten Gegenschließfläche 49 des Schaft-Innenrohres 22 und dem korrespondierenden kolbenseitigen Rand 50 vor. In Betätigungsstellung kommt es hingegen zu dem aus Fig. 11 ersichtlichen Schließen der Dosierkammer D.

Zur Erzielung der erläuterten Schleppkolben-Funktion sind der Boden 18 der Substanzen-Vorratskammer SV respektive der diese mitbildende Hohlraum 17 des Schaftes 15 und der Kolben 8 fest verbunden bzw. einstückig, dies unter Substituierung des als Hartteil realisierten Kolbenkopfes. Der geht praktisch im Gesamtgebilde auf.

Der so Teil der Kolbenmanschette stellende Boden 18 führt sich mit einer in Ausgaberichtung schräg gestellten Lippe 63 gleitend an der korrespondierenden Innenwand des Schaftes 15, welcher nun führungszylinderartige Aufgabe übernimmt. Der Kolben 8 steht dabei unter Reibschluß zwischen den Teilen 10 und 11.

An die spitzwinklig schräg gestellte Lippe 13 schließt der Boden 18 als Schrägwandung an. Die wurzelt dosierkammerbeabstandet im Rücken des Kolbens 8. Es ist so ein zentrierend wirkender Trichter bezüglich eines abrutschenden Zugangs der pulverförmigen Substanz erzielt. Der diesbezügliche Rücken des Kolbens 8 verläuft ebenflächig, d.h. senkrecht zur Längsmittelachse x-x des Inhalators 1.

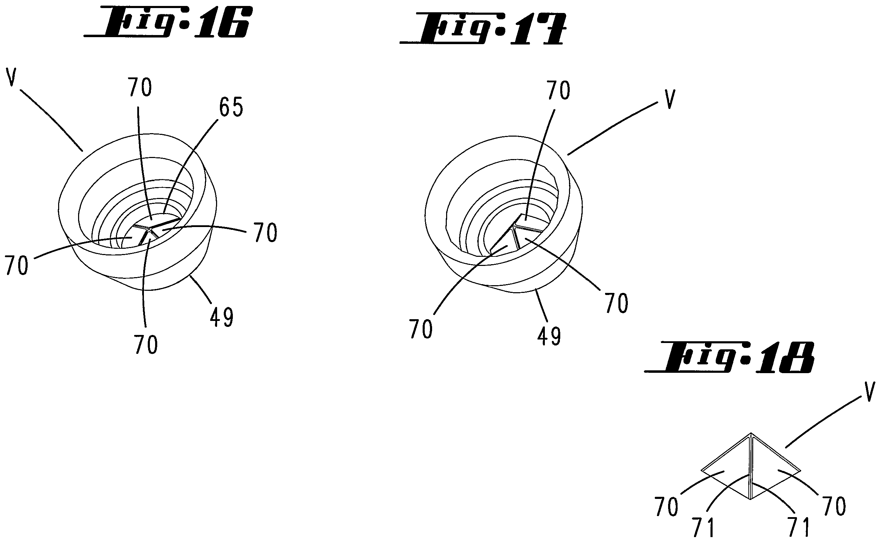

Der Teil des Bodens 18 bildende Kolben 8 weist auch hier bezüglich der Dosierkammer eine Vertiefung 45 auf. Oben, d.h. in Ausgaberichtung gesehen, endet diese Vertiefung 45 in den Rand 50, der mit der Gegenschließfläche 49 eines Ventilkegels 65 zusammenarbeitet. Letzterer ist Bestandteil eines aus Gummi oder dergleichen bestehenden Ventils V, welches auf das untere Ende a des Schaft-Innenrohres 22 rastierend aufgesteckt ist. Bezüglich des Ventilkegels 65 kann es sich tatsächlich um einen Kegel oder auch um einen pyramidalen Körper handeln. Die mehr kegelförmige Ausprägung ergibt sich anschaulich aus Figur 16, die eher pyramidale Ausformung hingegen lässt sich den Figuren 17 und 18 entnehmen. Das Ventil V überfängt die Bestandteil der Dosierkammer D stellende Vertiefung 45, wobei der Rauminhalt der hohlen Kegelseite als über die Ebene des Kolbenrückens hinausragende Ergänzung anschließt. Das Ventil V trägt so einen Überbau bei, welcher sich bis in die Spitze, aus dem ringförmigen Umfeld gespeist, anfüllt. Es findet gleichsam ein "Ausstanzen" der von der Vorratsmenge 20 separierten Ausgabemenge 20' statt. Die Abzweigung der letzteren ist abgeschlossen, wenn die Betätigung einsetzt, und zwar schon gleich zu Anfang bei geringfügig angefahrenem Hub, wie das aus Figur 11 hervorgeht. Bei diesem durch Abwärtsverlagern des Schaftes 15 stattfindenden Schließen schiebt das Ventil V den Kolben 8 reibungsschlüssig gebremst vor sich her. Der Widerstand - wie schon weiter oben angedeutet - ergibt sich über die Lippe 10, welche mit leichter Vorspannung über die Zylinderwand 11 gleitet. Wie ersichtlich, sind die Verhältnisse hinsichtlich der Bildung der Zylinderwand 11 hier wie zum zweiten Ausführungsbeispiel geschildert. Die Bezugsziffern sind sinngemäß angewandt. Der mit der Bodenplatte 4 integrale Stutzen 59 ist jedoch jetzt so gelegt, dass er mit seiner Mantelfläche eine periphere Federkammer 66 begrenzt, in welcher die Feder 12 geführt zwischen dem besagten Stutzen 59 und der Außenwand 67 des Gehäuses 2 unterkommt.

Lässt man den Schaft 15 los, treibt die Feder 12 den Schaft 15 in die aus Fig. 10 ersichtliche Grundstellung, in welcher der in einem Nacken 54 hier des Schaftes 15 unmittelbar gefesselte Ringkörper 53 durch einen Versprung des Gehäuses 2 anschlagbegrenzt wieder in die Rastnut 55 der Ansprechschwelle einschnäppert. Der Vorsprung ist die Schulter zwischen der Außenwand 57 und dem Hals 32.

Die Dosierkammer D ist offen und verfüllt sich auf diesem Wege mit pulverförmiger Substanz aufgrund des hinter dem Kolben 8 in der geschilderten Weise erzeugten Unterdrucks. Der Kolben 8 wird in Schleppposition gehalten. Seine den Leerhub LH nutzbar machende Relativverlagerung zum Schaft 15 ergibt sich durch den Eingriff eines Kragens 68 der Kolbenmanschette in einen entsprechend breiten Schlitz 69 am kolbenseitigen Ende der Innenwand des Schaftes 15. Die Mitnahme geschieht über die untere Flanke des besagten Schlitzes 69. Die obere Flanke des Schlitzes 69 steht in einem Abstand vom Rücken der bodenbildenden Kolbenmanschette, der größer ist als der axiale Leerhub LH. Es kann auch ein weggleicher Anschlag zwischen der geschilderte Stelle und zwischen Rand 50 und Gegenschließfläche 49 angewandt sein. Bevorzugt wird jedoch die Maßnahme zwischen dem Kragen 68 und dem Schlitz 69.

Die teilweise aus der Vertiefung 45 der Kolbenmanschette und teilweise aus der Höhlung des aufsetzenden Ventilkegels 65 gebildete Dosierkammer D füllt sich beim Aufsetzen des Ventils V bis in die sich verjüngende Spitze der Höhlung des Ventilkegels an. Der Schließdruck ist andererseits aber auch nicht geeignet, durch die zusammengedrückte Substanz zu öffnen; vielmehr bedarf es der geschilderten Betätigung des Gerätes.

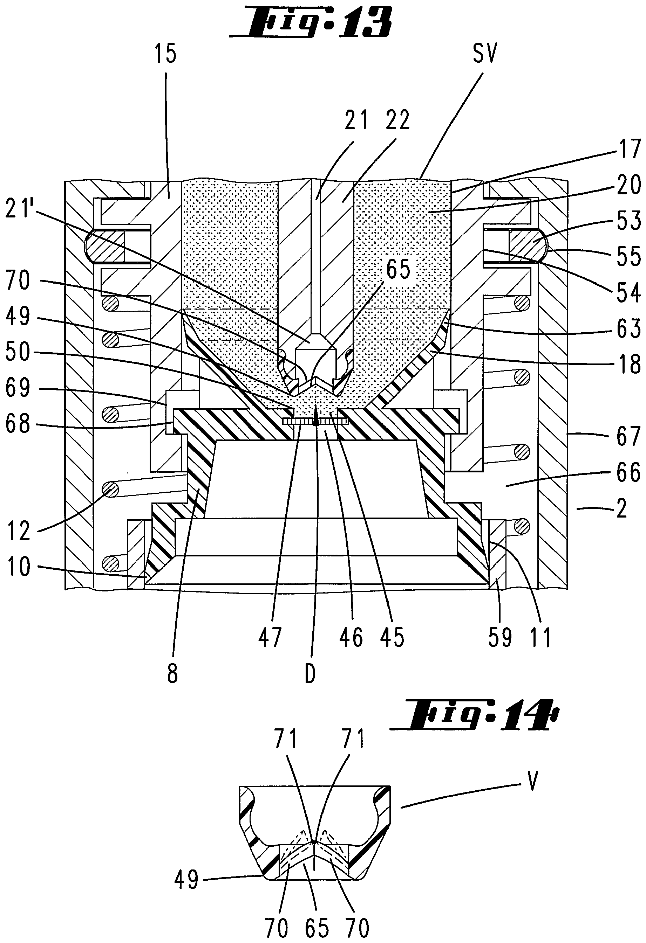

Hinsichtlich des Ventilkegels 65 bleibt noch zu erwähnen, dass dieser hinsichtlich seiner beispielsweise durch einen Kreuzschnitt geschaffenen, rückschlagventilartig wirkenden Ventillappen 70 zu einem stets sicheren Öffnungs- und Schließverhalten kommt. Ferner sind die Ventillappen 70 bezüglich ihrer in Ausgaberichtung weisenden, ausspitzenden Lippen 71 verdickt. Die Lippen 71 treten flächengrößer gegeneinander als die Dicke der Ventillappen 71 beträgt. Die Trennschnittstellen lassen sich formtechnisch entsprechend ergänzen. Zur Bildung der Lippen 71 kann der rückwärtige Bereich derselben bspw. ausgemuldet sein, wie das beispielsweise aus Fig. 14 hervorgeht.

Zur Schaffung eines ausreichenden Öffnungsfreiraumes für die Ventillappen 70 ist der sich in Ausgaberichtung Pfeil y erstreckende Abschnitt des Austragskanales 21 genügend weit zylindrisch ausgenommen, um im Anschluss an diese querschnittsgrößere Höhlung in den beschriebenen Trichter 21' überzugehen.

Die Steckmontage zwischen Kolben 8 und Schaft 15 ist erleichtert durch das von Hause aus elastisch gestellte Material des besagten Kolbens. Steckfördernde Maßnahmen in Form üblicher Auflaufschrägen können zusätzlich berücksichtigt sein (nicht dargestellt).

Das vorstehend als Inhalator bezeichnete Gerät, bei dem also grundsätzlich eine Substanz luftgetragen zerstäubt ausgegeben wird, kann auch zu anderen Einsatzformen verwendet werden, z.B. als Applikator, Pulverdosierer, Pulversprayer oder dergleichen, selbst als Zerstäuber auch für Farben und Gifte.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Handbetätigbarer Inhalator (1) für pulverförmige Substanzen, insbesondere medikamentöse Substanzen, bei welchem sich bei der Handbetätigung eine bestimmte Ausgabemenge (20') aus einer Substanzen-Vorratsmenge (20) in einer Dosierkammer (D) vor einem Austragskanal (21) abteilt zwecks luftgetragener Ausgabe aus einer Mundstücköffnung (14) am Ende (b) des Austragskanals (21), wobei ein den Ausgabe-Luftstrom erzeugender Kolben (8) mit einem Hohlraum (17) seines Schaftes (15) eine Substanzen-Vorratskammer (SV) und die Dosierkammer (D) bildet, wobei ein beim Rückhub des Kolbens (8) erzeugter Unterdruck die Dosierkammer (D) zur Substanzen-Vorratsmenge (20) hin öffnet, und wobei weiter der Boden der Dosierkammer (D) von einer luftdurchlässigen Membran gebildet ist, **dadurch gekennzeichnet, dass** das aus der Kolbenbewegung resultierende Luftstrom-Volumen mehr als das Hundertfache des Volumens der Dosierkammer (D) beträgt, aber weniger als das Sechshundertfache.

2. Inhalator nach Anspruch 1, **dadurch gekennzeichnet, dass** der Schaft (15) an einem dem Kolben (8) gegenüberliegenden Ende (b) die Mundstücköffnung (14) bildet.

3. Inhalator nach einem der vorhergehenden Ansprüche, **dadurch gekennzeichnet, dass** der Austragskanal (21) als sich im Zentrum des Schaftes (15) des unter Federspannung stehenden Kolbens (8) erstreckendes Schaft-Innenrohr (22) gestaltet ist, unter dessen kolbenseitigem Ende (a) sich die Ausgabemenge (20') sammelt.

4. Inhalator nach einem der vorhergehenden Ansprüche, **dadurch gekennzeichnet, dass** ein handbetätigter Kolben-Federspannhub ein Ausgabehub ist und sich die Ausgabemenge (20') bei einem federveranlassten Rückhub des Kolbens (8) sammelt.

5. Inhalator nach Anspruch 4, **dadurch gekennzeichnet, dass** sich die Ausgabemenge (20') in einer Vertiefung (45) im Boden (18) der Substanzen-Vorratskammer (SV) sammelt und der obere Rand (50) der Vertiefung (45) zwischen einer Dicht- und Öffnungsstellung des Schaft-Innenrohres wechselt.

6. Inhalator nach Anspruch 5, **dadurch gekennzeichnet, dass** ein Übertritt in die Öffnungsstellung aus einer elastischen Verlagerung des Bodens (18) der Substanzen-Vorratskammer (SV) aufgrund des beim Rückhub des Kolbens (8) hinter diesem auftretenden Unterdruckes erzielt ist.

7. Inhalator nach einem der Ansprüche 5 oder 6, **gekennzeichnet durch** eine mindestens in Richtung der Mundstücköffnung (14) luftdurchlässige Abdeckung (47) eines Loches (46) im Boden (18) der Substanzen-Vorratskammer (SV).

8. Inhalator nach einem der Ansprüche 5 bis 7, **dadurch gekennzeichnet, dass** der Boden (18) und die Vertiefung (45) der Substanzen-Vorratskammer (SV) von einer elastischen Membran gebildet sind, deren Topf-Innenwand ein Einsatzteil (48) trägt, auf dessen oberen Rand (50) das Schaft-Innenrohr (22) mit einer Gegenschließfläche (49) dichtend aufsetzt.

9. Inhalator nach einem der vorhergehenden Ansprüche, **dadurch gekennzeichnet, dass** der Kolben (8) eine entgegen der Richtung des Rückhubes weisende Kolbenlippe (10) besitzt, die gleitend an einer Innenwand (11) eines Zylinders (3) anliegt.

10. Inhalator nach einem der Ansprüche 7 bis 9, **dadurch gekennzeichnet, dass** die Durchlässigkeit der Abdeckung (47) in Relation zur Feinkörnigkeit des Pulvers so ist, dass eine nach erster Öffnungsbewegung des Bodens (18) auf diesen fallende dünnschichtige Pulvermenge die Luftdurchlässigkeit (24) in Öffnungsrichtung beseitigt.

11. Inhalator nach einem der Ansprüche 2 bis 10, **dadurch gekennzeichnet, dass** das Schaft-Innenrohr (22) sich bis kurz vor die Mundstücköffnung (14) erstreckt und zur Wand von umgebendem Schaftmaterial (23) einen Lufteinströmkanal freilässt, der bis in die Substanzen-Vorratskammer (SV) reicht.

12. Inhalator nach einem der Ansprüche 3 bis 11, **dadurch gekennzeichnet, dass** in einem oberen Bereich der Substanzen-Vorratskammer (SV) eine für die Einströmluft durchlässige Decke (19) vorgesehen ist, die das Schaft-Innenrohr (22) beiderseits abstützend kreuzt und ein zentrales Loch (40) fluchtend zum Austragskanal (21) besitzt.

13. Inhalator nach einem der vorhergehenden Ansprüche, **dadurch gekennzeichnet, dass** der Austragskanal (21) sich in Strömungsrichtung (Pfeil y) an einer Ausgabemengen-Sammelstelle trichterförmig (21') verjüngt.

14. Inhalator nach einem der Ansprüche 12 oder 13, **dadurch gekennzeichnet, dass** vor dem zentralen Loch (40) ein Ventilkörper (43) angeordnet ist, der in Austragsrichtung öffnet.

15. Inhalator nach einem der vorhergehenden Ansprüche, **gekennzeichnet durch** eine Ansprechschwelle für eine handbetätigte Kolbenverlagerung.

16. Inhalator nach Anspruch 15, **dadurch gekennzeichnet, dass** die Ansprechschwelle an einem Ringkörper (53) des Kolbenschaftes (15) rückseitig der Kolbenmanschette gebildet ist, welcher Ringkörper (53) in eine Rastnut (55) der dem Kolben (8) zugehörigen Zylinderwand (11) einrastet.

17. Inhalator nach einem der vorhergehenden Ansprüche, **dadurch gekennzeichnet, dass** die Dosierkammer (D) von einem endseitigen Verbreiterungsbereich des auf die luftdurchlässige Membran (47) aufsetzenden und beim Federrückhub abhebenden Austragskanal (21) gebildet ist.

18. Inhalator nach einem der vorhergehenden Ansprüche, **dadurch gekennzeichnet, dass** eine Umfassungswand (58) der Dosierkammer (D) sich in einer Austragsrichtung (Pfeil y) kegelstumpfförmig erweitert.

19. Inhalator nach Anspruch 18, **dadurch gekennzeichnet, dass** die Umfassungswand (58) der Dosierkammer (D) aus elastischem Material besteht und einstückig mit dem Boden (18) der Substanzen-Vorratskammer (SV) ausgebildet ist.

20. Inhalator nach einem der vorhergehenden Ansprüche, **gekennzeichnet durch** zwei beiderseits des zentralen Austragskanals (21) in einer gemeinsamen Ebene zu diesem verlaufende Lufteinströmkanäle (24) für die Substanzen-Vorratskammer (SV).

21. Inhalator nach Anspruch 20, **dadurch gekennzeichnet, dass** die Lufteinströmkanäle (24) in der Mittelebene zwischen taillenförmigen Ablageflächen-Einbuchtungen (26) liegen.

22. Inhalator (1) nach einem der vorhergehenden Ansprüche, **dadurch gekennzeichnet, dass** die Dosierkammer (D) in Grundstellung des Schaftes (15) zur Substanzen-Vorratskammer (SV) hin geöffnet ist.

23. Inhalator nach Anspruch 22, **dadurch gekennzeichnet, dass** sich die Dosierkammer (D) zur Substanzen-Vorratskammer (SV) hin durch einen Leerhub (LH) zwischen dem Schaft (15) und einer die Dosierkammer (D) formenden Kolbenmanschette öffnet.

24. Inhalator nach Anspruch 23, **dadurch gekennzeichnet, dass** ein Boden (18) der Substanzen-Vorratskammer (SV) Teil der Kolbenmanschette ist und an der Innenwand des Schaftes (15) gleitend anliegt.

25. Inhalator nach einem der Ansprüche 23 oder 24, **dadurch gekennzeichnet, dass** die Dosierkammer (D) teilweise aus einer Vertiefung (45) der Kolbenmanschette gebildet ist und teilweise aus einem sich dichtend auf den Rand (50) der Vertiefung (45) der Dosierkammer (D) aufsetzenden, in Ausgaberichtung verjüngenden Ventilkegel (65) besteht.

26. Inhalator nach einem der Ansprüche 23 bis 25, **dadurch gekennzeichnet, dass** der Leerhub (LH) federveranlasst und dadurch gebildet ist, dass ein Kragen (68) der Kolbenmanschette in einen entsprechend breiten Schlitz (69) an der Innenwand des Schaftes (15) ragt.

27. Inhalator nach einem der Ansprüche 23 bis 26, **dadurch gekennzeichnet, dass** eine Zylinderwand (11) für die Kolbenmanschette von einem Stutzen (59) einer Bodenplatte (4) gebildet ist.

28. Inhalator nach Anspruch 27, **dadurch gekennzeichnet, dass** eine Feder (12) sich außenseitig eines Stutzens (59) erstreckt, in einem Ringspalt zwischen dem Stutzen (59) und einer Außenwand (67) des Inhalators (1).

29. Inhalator nach einem der Ansprüche 25 bis 28, **dadurch gekennzeichnet, dass** der Ventilkegel (65) freigeschnittene, in Ausgaberichtung weisende Lippen (71) besitzt, deren Trennschnittenden verdickt sind.

## Claims

1. Manually actuable inhaler (1) for pulverulent substances, in particular medicinal substances, in which, during the manual actuation, a defined discharge quantity (20') from a substance storage quantity (20) is apportioned out in a metering chamber (D) upstream of a discharge passage (21) for the purpose of airborne discharge from a mouthpiece opening (14) at the end (b) of the discharge passage (21), wherein a piston (8) which generates the discharge airstream, together with a cavity (17) of its body portion (15), forming a substance storage chamber (SV) and the metering chamber (D), wherein a reduced pressure which is generated during the return stroke of the piston (8) opens the metering chamber (D) toward the substance storage quantity (20), and wherein furthermore the base of the metering chamber (D) being formed by an air-permeable membrane, **characterized in that** the airstream volume which results from the piston movement amounts to more than one hundred times but less than six hundred times the volume of the metering chamber (D).

2. Inhaler according to claim 1, **characterized in that** the body portion (15), at an opposite end (b) from the piston (8), forms the mouthpiece opening (14).

3. Inhaler according to one of the preceding claims, **characterized in that** the discharge passage (21) is configured as a piston body portion inner tube (22), which extends in the center of the body portion (15) of the piston (8) - the piston being under spring loading - and the discharge quantity (20') collecting beneath the piston-side end (a) of the inner tube.

4. Inhaler according to one of the preceding claims, **characterized in that** a manually actuated piston spring loading stroke is a discharge stroke and the discharge quantity (20') collects during a spring-triggered return stroke of the piston (8).

5. Inhaler according to claim 4, **characterized in that** the discharge quantity (20') collects in a recess (45) in the base (18) of the substance storage chamber (SV), and the upper edge (50) of the recess (45) alternates between a sealing position and an opening position of the piston body portion inner tube.

6. Inhaler according to claim 5, **characterized in that** a transfer into the opening position results from an elastic displacement of the base (18) of the substance storage chamber (SV) on account of the reduced pressure occurring behind the piston (8) during the return stroke of the latter.

7. Inhaler according to one of the preceding claims 5 or 6, **characterized by** a covering (47), which is air-permeable at least in the direction of the mouthpiece opening (14), of a hole (46) in the base (18) of the substance storage chamber (SV).

8. Inhaler according to one of claims 5 to 7, **characterized in that** the base (18) and the recess (45) of the substance storage chamber (SV) are formed by an elastic membrane, the cup inner wall of which carries an insert part (48), onto the upper edge (50) of which the piston body portion inner tube (22) touches down in a sealing manner by means of a mating closure surface (49).

9. Inhaler according to one of the preceding claims, **characterized in that** the piston (8) has a piston lip (10) which faces in the opposite direction to the direction of the return stroke and engages in a sliding manner against an inner wall (11) of a cylinder (3).

10. Inhaler according to one of claims 7 to 9, **characterized in that** the permeability of the covering (47) in relation to the fineness of the grains of the powder is such that the thin-layer powder quantity which drops onto the base (18) after a first opening movement of the latter eliminates the air permeability (24) in the opening direction.

11. Inhaler according to one of claims 2 to 10, **characterized in that** the piston body portion inner tube (22) extends to just before the mouthpiece opening (14) and leaves open, toward the wall of the surrounding piston body portion material (23), an air inflow passage which extends into the substance storage chamber (SV).

12. Inhaler according to of claims 3 to 11, **characterized in that** a cover (19), which is permeable to the inflow air, crosses the piston body portion inner tube (22) in a supporting manner on both sides and has a central hole (40) aligned with the discharge passage (21), is provided in an upper region of the substance storage chamber (SV).

13. Inhaler according to one of the preceding claims, **characterized in that** the discharge passage (21) narrows in a funnel shape (21') in the direction of flow (arrow y) at a discharge quantity collection location.

14. Inhaler according to one of claims 12 or 13, **characterized in that** a valve body (43), which opens in the discharge direction, is disposed in front of the central hole (40).

15. Inhaler according to one of the preceding claims, **characterized by** a response threshold for a manually actuated piston displacement.

16. Inhaler according to claim 15, **characterized in that** the response threshold is formed at an annular body (53) of the piston body portion (15) on the rear side of the piston sleeve, which annular body (53) latches into a latching groove (55) in the cylinder wall (11) belonging to the piston (8).

17. Inhaler according to one of the preceding claims, **characterized in that** the metering chamber (D) is formed by an end-side widening region of the discharge passage (21), which touches down onto the air-permeable membrane (47) and is lifted off during the spring return stroke.

18. Inhaler according to one of the preceding claims, **characterized in that** a boundary wall (58) of the metering chamber (D) widens frustoconically in discharge direction (arrow y).

19. Inhaler according to claim 18, **characterized in that** the boundary wall (58) of the metering chamber (D) consists of elastic material and is formed integrally with the base (18) of the substance storage chamber (SV).

20. Inhaler according to one of the preceding claims, **characterized by** two air inflow passages (24) for the substance storage chamber (SV), which run on both sides of the central discharge passage (21) in a common plane with the latter.

21. Inhaler according to claim 20, **characterized in that** the air inflow passages (24) are located in the center plane between waist-like supporting surface indentations (26).

22. Inhaler (1) according to one of the preceding claims, **characterized in that** the metering chamber (D), in the basic position of the piston body portion (15), is open toward the substance storage chamber (SV).

23. Inhaler according to claim 22, **characterized in that** the metering chamber (D) opens toward the substance storage chamber (SV) as a result of an idling stroke (LH) between the body portion (15) and a piston sleeve which forms the metering chamber (D).

24. Inhaler according to claim 23, **characterized in that** a base (18) of the substance storage chamber (SV) is part of the piston sleeve and rests in a sliding manner against the inner wall of the piston body portion (15).

25. Inhaler according to claim 23 or 24, **characterized in that** the metering chamber (D) is partially formed from a recess (45) in the piston sleeve and partially comprises a valve cone (65) which touches down in a sealing manner onto the edge (50) of the recess (45) in the metering chamber (D) and narrows in the discharge direction.

26. Inhaler according to one of claims 23 to 25, **characterized in that** the idling stroke (LH) is spring-triggered and is formed by virtue of a collar (68) of the piston sleeve projecting into a slot (69) of corresponding width on the inner wall of the piston body portion (15).

27. Inhaler according to one of claims 23 to 26, **characterized in that** a cylinder wall (11) for the piston sleeve is formed by a connection piece (59) of a baseplate (4).

28. Inhaler according to claim 27, **characterized in that** a spring (12) extends on the outside of a connection piece (59), in an annular gap between the connection piece (59) and an outer wall (67) of the inhaler (1).

29. Inhaler according to one of claims 25 to 28, **characterized in that** the valve cone (65) has lips (71) which are cut free and face in the discharge direction and the cut ends of which are thickened.

## Revendications

1. Inhalateur (1) à actionnement manuel pour substances pulvérulentes, en particulier des substances médicamenteuses, dans lequel, lors de l'actionnement manuel, une quantité à décharger donnée (20') est séparée d'une quantité de substances stockée (20) en la plaçant dans une chambre de dosage (D) en amont d'un canal de décharge (21) en vue de la décharge par transport par air hors d'une ouverture d'embout (14) à l'extrémité (b) du canal de décharge (21), dans lequel un piston (8) générateur du flux d'air de décharge forme avec une cavité (17) de sa jupe (15) une chambre de stockage de substances (SV) et la chambre de dosage (D), dans lequel une dépression générée lors de la course de retour du piston (8) ouvre la chambre de dosage (D) sur la quantité de substances stockée (20), et dans lequel en outre le fond de la chambre de dosage (D) est formée par une membrane perméable à l'air, **caractérisé en ce que** le volume de flux d'air résultant du mouvement de piston est supérieur à cent fois le volume de la chambre de dosage (D), mais en étant inférieur à six cents fois ce dernier.

2. Inhalateur selon la revendication 1, **caractérisé en ce que** la jupe (15) forme, à une extrémité opposée au piston (8), l'ouverture d'embout (14).

3. Inhalateur selon l'une des revendications précédentes, **caractérisé en ce que** le canal de décharge (21) est réalisé en tant que tube intérieur de jupe (22) s'étendant au centre de la jupe (15) du piston (8) placé sous contrainte élastique, la quantité de décharge (20') étant collectée sous l'extrémité côté piston (a) du tube intérieur de jupe (22).

4. Inhalateur selon l'une des revendications précédentes, **caractérisé en ce qu'**une course de mise sous tension élastique du piston actionné manuellement est une course de décharge et que la quantité à décharger (20') est collectée lors d'une course de retour du piston (8) suscitée élastiquement.

5. Inhalateur selon la revendication 4, **caractérisé en ce que** la quantité à décharger (20') est collectée dans une cavité (45) dans le fond (18) de la chambre de stockage de substance (SV) et que le bord supérieur (50) de la cavité (45) alterne entre une position de fermeture et une position d'ouverture du tube intérieur de jupe.

6. Inhalateur selon la revendication 5, **caractérisé en ce qu'**un passage dans la position d'ouverture est obtenu par un déplacement élastique du fond (18) de la chambre de stockage de substances (SV) en raison de la dépression générée derrière le piston (8) lors de sa course de retour.

7. Inhalateur selon l'une des revendications 5 ou 6, **caractérisé par** un couvercle (47) d'un trou (46) dans le fond (18) de la chambre de stockage de substances (SV) lequel couvercle (4) est perméable à l'air au moins en direction de l'ouverture d'embout (14).

8. Inhalateur selon l'une des revendications 5 à 7, **caractérisé en ce que** le fond (18) et la cavité (45) de la chambre de stockage de substances (SV) sont formés par une membrane élastique dont la paroi intérieure en forme de pot supporte un insert (48) sur le bord supérieur (50) duquel le tube intérieur de jupe (22) vient en appui étanche avec une surface de contre-fermeture (49).

9. Inhalateur selon l'une des revendications précédentes, **caractérisé en ce que** le piston (8) comprend une lèvre de piston (10) orientée à l'encontre de la direction de la course de retour, laquelle lèvre de piston (10) vient en appui glissant contre une paroi intérieure (11) d'un cylindre (3).

10. Inhalateur selon l'une des revendications 7 à 9, **caractérisé en ce que** la perméabilité du couvercle (47) en relation à finesse des particules de la poudre est telle qu'une quantité de poudre tombant en couche fine sur le fond (18) après un premier mouvement d'ouverture de celui-ci, supprime la perméabilité à l'air dans la direction de l'ouverture.

11. Inhalateur selon l'une des revendications 2 à 10, **caractérisé en ce que** le tube intérieur de jupe (22) s'étend jusqu'à juste avant l'ouverture d'embout (14) et laisse libre vers la paroi du matériau de jupe (23) qui l'entoure un canal d'admission d'air qui s'étend jusque dans la chambre de stockage de substances (SV).

12. Inhalateur selon l'une des revendications 3 à 11, **caractérisé en ce qu'**est prévu dans une région supérieure de la chambre de stockage de substances (SV), un couvercle (19) laissant passer le flux d'air entrant, lequel couvercle (19) croise le tube intérieur de jupe (22) en le supportant des deux côtés et comprend un trou central (40) aligné avec le canal de décharge (21).

13. Inhalateur selon l'une des revendications précédentes, **caractérisé en ce que** le canal de décharge (21) se rétrécit en forme d'entonnoir (21') dans la direction d'écoulement (flèche y) à l'endroit de collecte de la quantité à décharger.

14. Inhalateur selon l'une des revendications précédentes, **caractérisé en ce qu'**est agencé devant le trou central (40) une valve (43) qui ouvre dans la direction de décharge.

15. Inhalateur selon l'une des revendications précédentes, **caractérisé par** un seuil de réponse pour un déplacement du piston actionné manuellement.

16. Inhalateur selon la revendication 15, **caractérisé en ce que** le seuil de réponse est formé sur un élément annulaire (53) de la jupe de piston (15) du côté arrière du manchon de piston, lequel élément annulaire (53) est enclenché dans une rainure (55) de la paroi de cylindre (11) appartenant au piston (8).

17. Inhalateur selon l'une des revendications précédentes, **caractérisé en ce que** la chambre de dosage (D) est formée par une zone d'élargissement du côté extrémal du canal de décharge (21) lequel repose sur la membrane perméable à l'air (47) et se soulève lors de la course de retour élastique.

18. Inhalateur selon l'une des revendications précédentes, **caractérisé en ce qu'**une paroi extérieure (58) de la chambre de dosage (D) s'élargit de manière tronconique dans une direction de décharge (flèche y).

19. Inhalateur selon la revendication 18, **caractérisé en ce que** la paroi extérieure (58) de la chambre de dosage (D) est réalisée en matériau élastique et est formée d'un seul tenant avec le fond (18) de la chambre de stockage de substances (SV).

20. Inhalateur selon l'une des revendications précédentes, **caractérisé par** deux canaux d'admission d'air (24) pour la chambre de stockage de substances (SV) qui s'étendent de part et d'autre du canal de décharge central (21) dans un même plan avec celui-ci.

21. Inhalateur selon la revendication 20, **caractérisé en ce que** les canaux d'admission d'air (24) sont situés dans le plan médian entre des surfaces de support sous forme d'échancrures (26) formant taille.

22. Inhalateur (1) selon l'une des revendications précédentes, **caractérisé en ce que** la chambre de dosage (D) dans la position de base de la jupe (15) est ouverte sur la chambre de stockage de substances (SV).

23. Inhalateur selon la revendication 22, **caractérisé en ce que** la chambre de dosage (D) est ouverte sur la chambre de stockage de substances (SV) de par une course à vide (LH) entre la jupe (15) et un manchon de piston formant la chambre de dosage (D).

24. Inhalateur selon la revendication 23, **caractérisé en ce qu'**un fond (18) de la chambre de stockage de substances (SV) fait partie du manchon de piston et vient en appui glissant contre la paroi intérieure de la jupe (15).

25. Inhalateur selon l'une des revendications 23 ou 24, **caractérisé en ce que** la chambre de dosage (D) est formée en partie par une cavité (45) du manchon de piston et en partie par un clapet de valve (65) qui vient en appui étanche sur le bord (50) de la cavité (45) de la chambre de dosage (D) et qui se rétrécit dans la direction de décharge.

26. Inhalateur selon l'une des revendications 23 à 25, **caractérisé en ce que** la course à vide (LH) est suscitée élastiquement et est formée par le fait qu'une collerette (68) du manchon de piston s'étend dans une fente de largeur correspondante dans la paroi intérieure de la jupe (15).

27. Inhalateur selon l'une des revendications 23 à 26, **caractérisé en ce qu'**une paroi de cylindre (11) pour le manchon de piston est formée par un manchon (59) d'une plaque de fond (4).

28. Inhalateur selon la revendication 27, **caractérisé en ce qu'**un ressort (12) s'étend à l'extérieur d'un manchon (59) dans un espace annulaire entre le manchon (59) et une paroi extérieure (67) de l'inhalateur (1).

29. Inhalateur selon l'une des revendications 25 à 28, **caractérisé en ce que** le clapet de valve (65) présente des lèvres découpées (71) et orientées vers la direction de décharge et dont les extrémités découpées sont épaissies.
